# EUROPEAN PATENT SPECIFICATION

(11) **EP 4 125 802 B1**
(45) Date of publication and mention of the grant of the patent: **01.10.2025**
(21) Application number: 21714312.2
(22) Date of filing: 24.03.2021
(51) Int. Cl.: A61K 31/502, A61K 9/00, A61K 45/06, A61P 11/00, A61P 11/16, A61P 31/14

(54) **5-AMINO-2,3-DIHYDRO-1,4-PHTHALAZINEDIONE FOR TREATMENT OF ACUTE LUNG INJURY**
5-AMINO-2,3-DIHYDRO-1,4-PHTHALAZINDIONE ZUR BEHANDLUNG VON AKUTEN LUNGENVERLETZUNGEN
5-AMINO-2,3-DIHYDRO-1,4-PHTALAZINEDIONE POUR LE TRAITEMENT D'UNE LÉSION PULMONAIRE AIGUË

(30) Priority: 25.03.2020 EP 20000133
(43) Date of publication of application: 08.02.2023
(73) Proprietor: MetrioPharm AG, 8021 Zürich (CH)
(72) Inventor: BRYSCH, Wolfgang, 13505 Berlin (DE); KAISER, Astrid, 12305 Berlin (DE); SCHULZ, Petra, 13158 Berlin (DE); SCHUMANN, Sara, 14554 Seddiner See (DE); VON WEGERER, Jörg, 13597 Berlin (DE); SETZ, Christian, 96117 Memmelsdorf (DE); SCHUBERT, Ulrich, 07646 Trockenborn-Wolfersdorf (DE)
(74) Representative: Gruber, Daniel
(86) International application number: PCT/EP2021/000028
(87) International publication number: WO 2021/190783

(56) References cited:
- WO-A1-2007/018546
- WO-A1-2010/096686
- WO-A1-2017/140422
- WO-A1-2017/140430
- "MSL? Product Licensing Opportunity Non Confidential Summary", 15 April 2008 (2008-04-15), pages 1 - 13, XP055017086, Retrieved from the Internet <URL:http://www.bachpharma.com/images/LicensingSummary080415.pdf> [retrieved on 20120120]
- SCHUMANN SARA ET AL: "Immune-Modulating Drug MP1032 with SARS-CoV-2 Antiviral Activity In Vitro: A potential Multi-Target Approach for Prevention and Early Intervention Treatment of COVID-19", INTERNATIONAL JOURNAL OF MOLECULAR SCIENCES, vol. 21, no. 22, 20 November 2020 (2020-11-20), pages 8803, XP055781582, Retrieved from the Internet <URL:https://www.ncbi.nlm.nih.gov/pmc/articles/PMC7699954/pdf/ijms-21-08803.pdf> DOI: 10.3390/ijms21228803

## Description

The present application relates to the use of 5-amino-2,3-dihydro-1,4-phthalazinedione or one of its pharmaceutically acceptable salts in the prevention or treatment of acute lung injury. The invention in particular relates to the use of 5-amino-2,3-dihydro-1,4-phthalazinedione sodium salt for said purposes.

### BACKGROUND OF THE INVENTION

In early 2020 a pandemic with the coronavirus SARS-CoV-2 started to fledge the world. While most infections show a relatively mild disease course ca. 15 % of the diagnosed patients develop serious symptoms, mostly a severe pneumonia. In ca. 3 % of the diagnosed patients there is a lethal outcome. The most endangered patient group are the elderly and persons with a previous serious disease, e.g. when they are immunocompromised. The final stage of the disease is acute lung injury.

Acute lung injury (ALI) is a clinical syndrome of acute respiratory failure with substantial morbidity and mortality. It is characterized by an acute onset of bilateral pulmonary infiltrates with hypoxemia without evidence of hydrostatic pulmonary edema. For diagnosis, the American-European Consensus Conference Committee elaborated a consensus definition for ALI. This definition requires the acute onset of diffuse bilateral pulmonary infiltrates by chest radiograph; a PaO₂/FiO₂ (partial arterial pressure of oxygen / fractional concentration of oxygen in inspired air) ≤ 300 mm Hg and a pulmonary artery wedge pressure (PAWP) ≤ 18 mm Hg or no clinical evidence of left atrial hypertension (Bernard et al. (1994) J Crit Care 9: 72-81). The incidence of ALI in the USA is estimated to be between 64 and 86 / 100,000 persons and year. A mortality risk of 29 - 42 % was described (cf. Erickson et al. (2009) Crit Care Med 37: 1574-1579).

ALI is an acute inflammation leading to a disruption of the lung endothelial and epithelial barriers. Cellular characteristics of ALI include loss of alveolar-capillary membrane integrity, excessive transepithelial neutrophil migration, and release of pro-inflammatory, cytotoxic mediators (cf. Matthay and Zimmerman (2005) Am J Respir Cell Mol Biol 33: 319-327). Persistently elevated plasma levels of interleukin (IL)-6, IL-8, and tumor necrosis factor (TNF)-alpha are strongly predicative of mortality (Meduri et al. (1995) Chest 108: 1303-1314).

Microvascular endothelial injury leads to increased capillary permeability. This alteration in permeability permits the efflux of protein-rich fluid into the peribronchovascular interstitium, ultimately crossing the epithelial barrier into the distal airspaces of the lung (Pugin et al. (1999) Crit Care Med 27: 304-312). Excessive and/or prolonged activation of neutrophils contributes to basement membrane destruction and increased permeability of the alveolar-capillary barrier. Migrating groups of neutrophils result in the mechanical enlargement of paracellular neutrophil migratory paths. Neutrophils also release damaging proinflammatory and pro-apoptotic mediators that act on adjacent cells to create ulcerating lesions (Zemans et al. (2009) Am J Respir Cell Mol Biol 40: 519-535).

Alveolar epithelial type II cell injury also leads to a loss of surfactant production (Greene et al. (1999) Am J Respir Cell Mol Biol 160: 1843-1850) decreasing overall pulmonary compliance. Type II epithelial cells normally drive the epithelial repair process. Loss of this function can lead to disorganized, fibrosing repair (Bitterman (1992) Am J Med 92: 39S-343S).

Resolution of ALI is primarily dependent on a timely and orderly repair of the alveolar gas exchange apparatus. For gas exchange to improve, alveolar fluid transport must be upregulated, clearing the airspace of protein-rich edema fluid, and restoring the normal secretion of surface-active material from alveolar type II cells (Matthay and Zimmerman (2005) Am J Respir Cell Mol Biol 33: 319-327).

Treatment options of ALI include both assisted ventilation and pharmacological treatment. Still the best outcome has improved ventilator management.

Numerous potential pharmacologic treatments have been investigated until now. However, the use of exogenous surfactant, inhaled nitric oxide, intravenous prostaglandin E1, glucocorticoids, ketoconazole, lisofylline, N-acetylcysteine, and activated protein C showed mostly disappointing results (for review see Johnson and Matthay (2010) J Aerosol Medicine and Pulmonary Drug Delivery 23: 243-252). Beta-2 agonists accelerate the resolution of pulmonary edema by decreasing inflammation and upregulating alveolar salt and water transport, hastening the resolution of alveolar edema. However, a large multicenter randomized clinical trial of an aerosolized albuterol was stopped for futility (Matthay et al. (2009) Am J Respir Cell Mol Biol 179: A2166). Intravenously administered salbutamol significantly lowered extravascular lung water (Perkins et al. (2006) Am J Respir Cell Mol Biol 173: 281-287).

Statins are normally used for the prevention or treatment of cardiovascular diseases, but they also possess significant anti-inflammatory, immunomodulatory, and antioxidant effects. Statin users were found to have a decreased severity of sepsis and mortality despite having higher baseline comorbidities (cf. Thomsen et al. (2008) Arch Intern Med 168: 2081-2087).

In mice previously instilled with *E. coli* endotoxin bone-marrow derived mesenchymal stem cells (MSCs) decreased extravascular lung water, alveolar-capillary permeability and overall mortality (Gupta et al. (2007) J Immunol 179: 1855-1863).

A further treatment option is extracorporeal membrane oxygenation [ECMO] (cf. Freed et al. (2010) Can J Anaesth 57: 240-247).

WO 2017/140430 describes a polymorph crystalline form of 5-amino-2,3-dihydro-1,4-phthalazinedione. WO 2017/140422 reveals a method of production of another polymorph crystalline form of 5-amino-2,3-dihydro-1,4-phthalazinedione. An inhalatory administration of 5-amino-2,3-dihydro-1,4-phthalazinedione for the treatment of lung diseases is proposed in both patent applications. None of these applications, however, discloses or suggests a treatment with 5-amino-2,3-dihydro-1,4-phthalazinedione in acute lung injury, in particular caused by a coronaviral infection.

WO 2007/018546 mentions a possible use of phthalazinediones, among them of 5-amino-2,3-dihydro-1,4-phthalazinedione, in the treatment of Hantavirus pulmonary syndrome.

The company Bach Pharma proposes a possible use of 5-amino-2,3-dihydro-1,4-phthalazinedione in the treatment of respiratory inflammation, but not in acute lung injury or a coronaviral infection (www.bachpharma.com/images/licensingsummary080415.pdf, retrieved on 20.01.2012).

WO 2010/096686 discloses the use of 5-amino-2,3-dihydro-1,4-phthalazinedione in the treatment of intestinal diseases related to oxidative stress in HIV-infected persons, but it does not reveal a use in acute lung injury.

Delivery of pharmaceutically active agents via aerosol to the distal air spaces of the lung remains a promising option for both small molecules and proteins. Such a dosage form may be also applied in mechanically ventilated patients.

Thus, there is a medical need to find a pharmaceutically active agent that addresses inflammation in acute lung injury. Ideally, it should reduce the mortality rate, accelerate the recovery rate and the days a patient has to spend in an intensive care unit. Particularly in the light of the ongoing SARS-CoV-2 pandemic the latter is an essential feature because there is only a limited number of intensive care unit beds, respectively assisted ventilation machines available. These places soon get outnumbered by an unprecedented number of patients with acute lung injury.

Surprisingly, this task is solved by the administration of 5-amino-2,3-dihydro-1,4-phthalazinedione or one of its pharmaceutically acceptable salts or solvates, hydrates, crystalline polymorphs, tautomers or isotopically enriched forms thereof.

Thus, the present application discloses 5-amino-2,3-dihydro-1,4-phthalazinedione or one of its pharmaceutically acceptable salts for use in the prophylaxis or treatment of acute lung injury. The invention is limited to the appended claims.

### DETAILED DESCRIPTION OF THE INVENTION

5-amino-2,3-dihydro-1,4-phthalazinedione belongs to the pharmaceutical class of the phthalazinediones. Compounds of this class are known for their beneficial anti-inflammatory action. 5-amino-2,3-dihydro-1,4-phthalazinedione is also known under the name luminol. Luminol has excellent chemiluminescent properties. It is widely applied in diagnostic assays as a detection means and in forensic medicine, for example for tracing blood spots. In medicine, 5-amino-2,3-dihydro-1,4-phthalazinedione has been developed in the form of a sodium salt. In some countries it is approved for a broad range of acute and chronic inflammatory disorders, including a.o. acute infections of bacterial and viral origin, particularly of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. Further, there is still a long list of indications in scientific and patent literature in the treatment of which 5-amino-2,3-dihydro-1,4-phthalazinedione sodium salt was allegedly tested or a beneficial use was suggested (cf. WO 2004/041169; WO 2007/018546; WO 2012/127441; WO 2017/202496; WO 2018/082814: a.o.).

While most conventional immunomodulatory drugs show serious adverse reactions, or are at least problematic in long-term treatment, 5-amino-2,3-dihydro-1,4-phthalazinedione and its pharmaceutically acceptable salts are well tolerated and have a high safety margin in respect to administered dosages.

To ensure a better solubility and bioavailability pharmaceutically acceptable salts of 5-amino-2,3-dihydro-1,4-phthalazinedione are used. Sodium, potassium and lithium salts have been described for therapeutic applications (cf. WO 2010/082858). Crystal structures for lithium, sodium, potassium, rubidium and cesium salts were described in Guzei et al. (2013) Journal of Coordination Chemistry 66, 3722-3739. Thus, the present patent application refers also to the use of all pharmaceutically acceptable salts of 5-amino-2,3-dihydro-1,4-phthalazinedione. In particular, the present application discloses 5-amino-2,3-dihydro-1,4-phthalazinedione or one of its pharmaceutically acceptable salts for use in the prophylaxis or treatment of acute lung injury, wherein the pharmaceutically acceptable salt of 5-amino-2,3-dihydro-1,4-phthalazinedione is 5-amino-2,3-dihydro-1,4-phthalazinedione sodium salt.

5-amino-2,3-dihydro-1,4-phthalazinedione is often used as a hydrate, for example as sodium salt dihydrate. Thus, the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione or one of its pharmaceutically acceptable salts may build complexes with suitable ligands. Thus, the present patent application refers also to such complexes.

In order to ensure a reproducible and standardized API production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and WO 2016/096143 (Form III). These crystalline polymorphs are virtually free from phase impurities and were characterized by means of X-ray powder diffraction. This method yields a set of characteristic d-values indicating interplanar spacings [Å] and of the corresponding 2-theta (2θ) angles [°] under which Bragg reflections occur. This yields a unique and unambiguous fingerprint of the respective polymorphs.

For Form I the following values were determined:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3.

Form II is characterized by the following values:
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8.

Form III yielded the following values:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93.
5-amino-2,3-dihydro-1,4-phthalazinedione itself shows also polymorphism. A Form I (Paradies (1992) Ber. Bunsen-Ges. Phys. Chem 96: 1027-1031) and a Form II (WO 2017/140430) have been disclosed.

Thus, the present patent application refers also to the use according to the invention of all crystalline forms and polymorphs thereof of 5-amino-2,3-dihydro-1,4-phthalazinedione and its pharmaceutically acceptable salts.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the invention.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione (Proescher and Moody (1939) J Lab Clin Med, 1183-1189). Thus, the present patent application refers also to the use of all tautomers of 5-amino-2,3-dihydro-1,4-phthalazinedione and its pharmaceutically acceptable salts.

Isomer is a generic term for molecules with the same chemical formula but a different chemical structure. They can be differentiated into constitutional (structural) isomers (wherein an exchange of atoms or of a functional group occurs) and stereoisomers. Stereoisomers can be subdivided into enantiomers (non-superimposable mirror images of the same molecule) and diastereomers (the same molecule with a different configuration at one or more stereocenters). Diastereomers can be subdivided into cis/trans isomers (referring to the relative orientation of functional groups within a molecule) and on the other hand conformers (rotation about formally single bonds) and rotamers (different rotational positioning about a single bond). An example for a constitutional isomer of 5-amino-2,3-dihydro-1,4-phthalazinedione is 6-amino-2,3-dihydrophthalazine-1,4-dione (isoluminol). Stereoisomers may occur in phthalazinedione derivatives. Thus, the present patent application refers also to the use of all isomers of 5-amino-2,3-dihydro-1,4-phthalazinedione, its derivatives and pharmaceutically acceptable salts.

For some applications it may be desirable that isotopically enriched forms of the compounds of the invention are used, e.g. for diagnostic purposes. Thus, the present patent application refers also to such isotopically enriched forms of the compounds of the invention.

From a pharmacokinetic point of view or for a production rationale it may be preferable to use a prodrug as a dosage form. A prodrug is administered in a pharmacologically inactive form and is metabolically converted into the active form inside the body. This conversion may occur systemically or locally. Thus, the present patent application refers also to prodrugs of the compounds of the invention.

As used throughout the present application the term "5-amino-2,3-dihydro-1,4-phthalazinedione or one of its pharmaceutically acceptable salts" shall encompass all the aforementioned molecular variants of 5-amino-2,3-dihydro-1,4-phthalazinedione, i.e. 5-amino-2,3-dihydro-1,4-phthalazinedione or one of its pharmaceutically acceptable salts or solvates, hydrates, crystalline polymorphs, tautomers or isotopically enriched forms thereof.

Unless otherwise stated, any technical or scientific term used in the present invention has the meaning that a man skilled in the relevant technical art will attribute to them.

According to the application the terms "drug substance", "active substance", "active agent", "pharmaceutically active agent", "active ingredient" or "active pharmaceutical ingredient" (API) refer to 5-amino-2,3-dihydro-1,4-phthalazinedione or its pharmaceutically acceptable salts, if not stated otherwise or used in a general sense.

The terms "composition" or "pharmaceutical composition" comprise at least one active ingredient in any pharmacologically acceptable defined dosage and dosage form together with at least one pharmaceutically acceptable excipient, as well as all agents that are generated from the ingredients as outlined below directly or indirectly as a combination, accumulation, complex or crystal, or as a consequence of other reactions or interactions, as well as optionally at least one further pharmaceutical drug, as listed below.

The term "excipient" is used in this application to describe any component of a pharmaceutical composition apart of the pharmaceutically active principle. The selection of suitable excipients depends on a variety of factors, such as the dosage form, the dosage, the desired solubility and the stability of the composition.

The terms "effect", "therapeutic effect", "action", "therapeutic action", "efficacy" and "effectiveness" with regard to the substance of the invention or any other active substance mentioned in the description refers to causally occurring beneficial consequences in the organism to which said substance has been administered before.

According to the invention the terms "effective amount" and "therapeutically effective amount" refer to an amount of the substance of the invention that is sufficiently large to cause a desired beneficial effect in a subject in need of such a treatment.

The terms "treatment" and "therapy" comprise the administration of at least the substance of the invention, alone or in combination with at least one further pharmaceutical drug, independently of the chronological order of the administration. Such an administration is intended to substantially improve the disease course of acute lung injury by either completely curing the disease or by stopping or decelerating the increase of disabilities during course of the disease.

The terms "prophylaxis" or "prophylactic treatment" comprise the administration of at least the substance of the invention, alone or in combination with at least one further pharmaceutical drug, independently of the chronological order of the administration, in order to prevent or suppress the manifestation of symptoms attributed to acute lung injury. In particular, it refers to medical conditions of a patient in which the manifestation of such symptoms is expected to occur in the far or near future with a reasonable probability.

The terms "subject" and "patient" comprise individuals suffering from disease symptoms or disabilities related to acute lung injury wherein said diagnosis is either approved or suspected. Individuals are mammals, in particular humans.

In the scope of the present application the term "medicine" shall comprise human and veterinary medicine.

In the sense of the present patent application the terms "inflammatory disease" or "inflammatory pulmonary disease" refer to diseases, disorders or other body conditions in which an inflammation, in particular of the lungs, becomes manifest as a major symptom. An inflammation is the response of body tissues to irritation (exogenous or endogenous noxae) or injury. It can be provoked amongst others by physical, chemical and biologic stimuli, comprising mechanical trauma, radiation damage, corrosive chemicals, extremes of heat or cold, infectious agents such as bacteria, viruses, fungi and other pathogenic microorganisms or parts of them. An inflammation can have beneficial (e.g. within the scope of wound healing) and/or detrimental effects in the affected tissue(s). This may even lead to a loss of function of the affected tissue.

An inflammation is one of the first responses of the immune system that has become activated e.g. by an infection or degenerated endogenous cells. The system of innate immunity mediates an unspecific response, amongst others a general inflammatory response, while the adaptive immune system provides reactions specific to the respective pathogen, which will then be remembered by the immune system. An organism can be in an immunodeficient state, i.e. the immune response is not able to cope with the aforementioned irritations or injuries in a satisfactory manner. On the other hand, the immune system might become hyperactive and turn its defense against endogenous tissues, as in the case of autoimmune diseases.

If it is known that a healthy person is or will be vulnerable to develop acute lung injury, e.g. ahead of a surgery because a tissue damage is to be expected, or because the danger of an exposition to bacteria or viruses that may cause acute lung injury is unavoidable, e.g. for medical staff or scientific staff in respective laboratories, it can be indicated to give a prophylactic medication in order to prevent or at least to mitigate the expected impairment or damage. Thus, the present patent application refers also to a prophylactic use according to the invention.

In the scope of the present application the term "pulmonary" refers to organs and tissues of the lower respiratory tract. Examples of organs and tissues of the lower respiratory tract are, without being limiting, the lungs including their lobes, apices, lingulae and alveoli; the bronchi including respiratory bronchioles; tracheal and bronchi rings including the carina; pulmonary vessels including lung vessels and bronchial vessels and bronchial vessels; bronchopulmonary lymph nodes; autonomous nervous system of the lung.

In the scope of the present application the term "pulmonary" further refers to adjacent organs and tissues that functionally or structurally are closely linked to the lower respiratory tract and/or the thorax and therefore can be pharmaceutically accessed excellently by inhalation. Examples are, without being limiting, pleura and diaphragm.

In the scope of the present application the terms "alveoli" and "alveolar" refer to the tissue structures at the bottom of the lung airways. Alveoli are hollow cup-shaped cavities found in the lung parenchyma where gas exchange takes place. Further, they are located sparsely on the respiratory bronchioles, line the walls of the alveolar ducts, and are more numerous in the blind-ended alveolar sacs. The alveolar membrane is the gas exchange surface, surrounded by a network of capillaries. Across the membrane oxygen is diffused into the capillaries and carbon dioxide released from the capillaries into the alveoli to be breathed out. Alveoli consist of an epithelial layer of simple squamous epithelium and an extracellular matrix surrounded by capillaries. The epithelial lining is part of the alveolar membrane, also known as the respiratory membrane.

Type I and type II pneumocytes are found in the alveolar wall. Alveolar macrophages are immune cells that move about in the alveolar lumen and in the connective tissue between them. Type I cells are squamous epithelial cells, thin and flat and form the structure of the alveoli. Type II cells (goblet cells) release pulmonary surfactant to lower surface tension.

A typical pair of human lungs contain about 300 million alveoli, producing 70 m² of surface area. Each alveolus is wrapped in a fine mesh of capillaries covering about 70% of its area. The diameter of a typical healthy alveolus is between 200 and 500 µm.

The administration of 5-amino-2,3-dihydro-1,4-phthalazinedione to a patient suffering from acute lung injury leads to a dose-dependent reduction of ROS/RNS levels in neutrophils and macrophages, their migration through the lung epithelium is slowed down and the development of a pulmonary edema is suppressed or at least mitigated. This leads to a clearly higher survival rate of ALI patients in intensive care, the recovery time for the surviving patients is clearly reduced and hence the number of days a patient needs an intensive care place and/or assisted ventilation.

The use of 5-amino-2,3-dihydro-1,4-phthalazinedione sodium salt is preferred. The use of 5-amino-2,3-dihydro-1,4-phthalazinedione sodium salt Form I is particularly preferred.

Severe infections are the most common cause for ALI. They account for nearly 50% of the cases. These infections may cause pneumonia or systemic diseases such as sepsis, sepsis syndrome and septic shock (Piantadosi and Schwartz (2004) Ann Intern Med 141: 460-470). Viral pulmonary infections causing ALI can be either due to respiratory viruses or nosocomial viral infections with *Herpesviridae* such as *Herpes simplex* virus (HSV) and *Cytomegalovirus* (CMV) (cf. Luyt et al. (2008) Curr Opin Crit Care 14: 605-608).

Bacterial pneumonia that can aggravate to ALI is due to infections with gram-positive bacteria such as *Streptococcus pneumoniae* (*Pneumococcus*)*, Staphylococcus aureus* and *Bacillus anthracis,* gram-negative bacteria such as *Haemophilus influenzae, Klebsiella pneumoniae, Escherichia coli, Pseudomonas aeruginosa, Bordetella pertussis* and *Moraxella catarrhalis* as well as atypical bacteria such as *Coxiella burnetii, Chlamydophila pneumoniae, Mycoplasma pneumoniae, Legionella pneumophila, Serratia marcescens* and *Yersinia pestis.*

Sepsis, septic syndrome and septic shock are due to infections with gram-negative bacteria such as *Streptococcus pneumoniae, Staphylococcus aureus, Methicillin-resistant Staphylococcus aureus* (MRSA), *Acinetobacter baumannii*, *Yersinia ssp., Salmonella spp., Burkholderia pseudomallei, Francisella tularensis, Rickettsia sp., Neisseria gonorrhoeae* (*Gonococcus neissen*) and *Neisseria meningitidis* (*Meningococcus*), Gram-positive bacteria such as *Haemophilus influenzae, Pseudomonas aeruginosa, Bacillus anthracis, Listeria sp., Erysipelothrix ssp., Clostridium perfringens, Actinomyces ssp.* and *Escherichia coli,* as well as atypical bacteria such as *Legionella ssp.*

In some cases, such a septic condition can also be caused by a fungal infection, e.g. with *Candida sp., Aspergillus sp., Histoplasma sp.* or *Pneumocystis jirovecii.*

The present application discloses also 5-amino-2,3-dihydro-1,4-phthalazinedione or one of its pharmaceutically acceptable salts for use in the prophylaxis or treatment of acute lung injury, wherein the acute lung injury has been caused by a viral infection.

The most frequent class among respiratory viruses causing ALI are influenza viruses and rhinoviruses, followed by parainfluenza viruses, adenoviruses, respiratory syncytial virus, coronaviruses and human metapneumovirus (cf. Luyt et al. (2008) Curr Opin Crit Care 14: 605-608).

Examples for such Influenza A viruses include as Spanish influenza virus H1N1, influenza virus H1N8, influenza virus H2N1, Asian influenza virus H2N2, Hong Kong influenza virus H3N2 and avian influenza virus H5N1, swine influenza virus H9N2 and influenza virus H10N8.

Examples for exacerbated infections with commonly innocuous rhinoviruses include infections with enterovirus-human rhinovirus (EV-HRV) and human rhinoviruses A, B and C. Particularly children can be affected and need to be admitted to PICU (pediatric intensive care unit).

Human parainfluenza viruses endanger mostly small children. Examples include human parainfluenza viruses types 1 - 4 (HPIV 1-4).

Symptoms of adenovirus infections reach from a simple cold over acute bronchitis to pneumonia. Particularly immunocompromised persons are endangered to develop ALI. Such severe symptoms after an adenoviral infection are mainly caused by subgroups B (serum types HAdV-3, -7, -11, -14, -16, -21, -34, -35, -50, -55) and C (serum types HAdV-1, 2, 5, 6, 57).

Respiratory syncytial virus (RSV, HRSV) with its subtypes A and B affects mainly children and the elderly and can become critical. 2-3% of all infected children need to be treated in intensive care (cf. Hall et al. (2009) New England J Med 360: 588-598).

Human metapneumovirus (HMPV) is genetically similar to RSV and develops similar clinical symptoms. In particular small children are affected.

Infections with *Herpesviridae* can also lead to severe symptoms such as ALI. In humans 9 *Herpesviridae* are known: *Herpes simplex* viruses 1 and 2 (HSV-1 and HSV-2, or HHV1 and HHV2), *Varicella zoster* virus (VZV or HHV-3), *Epstein-Barr virus* (EBV or HHV-4), *human cytomegalovirus* (HCMV or HHV-5), human herpesvirus 6A and 6B (HHV-6A and HHV-6B), human herpesvirus 7 (HHV-7) and Kaposi's sarcoma-associated herpesvirus (KSHV, or HHV-8) (cf. Carter and Saunders (2007) Virology, Principles and Applications. John Wiley & Sons). Particularly HSV-1, HSV-2 and HCMV can cause serious pulmonary problems if the infection gets out of control (cf. Luyt al. (2011) Presse Med 40: e561-e568).

Infections with Hantaviruses include infections may cause Hantavirus pulmonary syndrome (HPS) that may end in ALI. Example are infections with *Orthohantavirus Hantaan* (HTNV), *Puumala Orthohantavirus* (PUUV), *Dobrava-Belgrade Orthohantavirus* (DOBV), *Seoul Orhohantavirus* (SEOV), *Sin Nombre Orthohantavirus* (SNV), *Black Creek Canal Orthohantavirus* (BCCV), *Mononhagela* Virus (MGLV), *New York Orthohantavirus* (NYV), *El Moro Canyon Orthohantavirus, Bayou Orthohantavirus* (BAYV), *Choclo Orthohantavirus* (CHOV) and *Andes Orthohantavirus* (ANDV).

Coronaviruses pathogenic in humans are SARS-CoV, SARS-CoV-2, MERS-CoV and HCoV-HKU1, HCoV-NL-63, HCoV-OC43 and HCoV-229E. The last four cause only relatively mild symptoms (cf. Andersen et al.: The Proximal Origin of SARS-CoV-2, on virologica.org, as of February 17th, 2020) while infections with SARS-CoV, SARS-CoV-2 or MERS-CoV bear a relatively high risk to lead to ALI.

In particular, the present application discloses thus 5-amino-2,3-dihydro-1,4-phthalazinedione or one of its pharmaceutically acceptable salts for use in the prophylaxis or treatment of acute lung injury, wherein acute lung injury is caused by a viral infection and said viral infection is a coronaviral infection by SARS-CoV, SARS-CoV-2 or MERS.

ALI can also be iatrogenic. Such adverse drug reactions were described e.g. for busulfan, bleomycin, methotrexate, azathioprine, BCNU (bis-chloroethyl-nitroso urea; carmustin), cytoxan, melphalan, mitomycin C, amiodarone, gold, nitrofurantoin, hexamethonium, placidly, penicillamine and intravenous administration of contrast material.

ALI can also be a severe complication of other diseases such as connective tissue disease, systemic lupus erythematosus, rheumatoid arthritis, polymyositis / dermatomyositis, scleroderma, mixed connective disease, pulmonary hemorrhage syndrome and vasculitis, Goodpasture syndrome, microscopic polyangiitis, polyarteritis nodosa, granulomatosis with polyangiitis, vasculitis associated with collagen vascular disease, acute pancreatitis, molar pregnancy, uremia and leukemic cell lysis.

ALI can also be caused by ingestants such as paraquat, kerosene, denatured rapeseed oil or by acute massive aspiration as well as by inhalants such as oxygen, amitrole-containing herbicide, ammonia and bleach mixture, hydrogen sulfide, mercury vapor, nitric acid fumes, nitrogen dioxide, paint remover, smoke, smoke bomb, sulfur dioxide and war gases.

ALI can be caused by shock, toxic shock syndrome, trauma, hemorrhages, radiation exposure, including via radiation-impregnated embolization beads, cardiopulmonary bypass, transfusion therapy, heat, burn, scald, near drowning, peritoneal-venous shunt, postlymphangiography, venous air embolism and high altitude. It can also be neurogenic or cardiogenic.

5-amino-2,3-dihydro-1,4-phthalazinedione or one of its pharmaceutically acceptable salts, a composition according to the invention or a drug combination according to the invention can be applied in the prophylaxis or treatment of acute lung injury by any medically acceptable administration route to a patient in need thereof. Such medically acceptable administration routes can be e.g. by inhalation, by intubation, orally, parenterally, intraperitoneally, intravenously, intraarterially or sublingually.

Preferred oral formulations for use in the prophylaxis or treatment of acute lung injury are capsules or tablets containing 5-amino-2,3-dihydro-1,4-phthalazinedione or one of its pharmaceutically acceptable salts in an amount of 50 mg, 100 mg, 150 mg, 200 mg, 300 mg, 400 mg, 500 mg or 600 mg, preferably 100 mg, 150 mg, 200 mg, 300 mg or 400 mg, most preferably 300 mg.

In another aspect of the invention a composition for use in the prophylaxis or treatment of acute lung injury is disclosed, wherein said composition contains 5-amino-2,3-dihydro-1,4-phthalazinedione or one of its pharmaceutically acceptable salts, a carrier and at least one pharmaceutically acceptable excipient.

The term "pharmaceutically acceptable excipient(s)" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process. Advantageous classes of excipients according to the invention include carriers, binding agents, colorants, buffers, preservatives, antioxidants, coatings, sweeteners, thickening agents, pH-regulators, acidity regulators acidifiers, solvents, isotonizing agents, disintegrants, glidants, lubricants, emulsifiers, solubilizing agents, stabilizers, diluents, anti-caking agents (antiadherents), sorbents, foaming agents, anti-foaming agents, opacifiers, fatliquors, consistency enhancers, hydrotropes, aromatic and flavoring substances.

In general, one or more pharmaceutically acceptable carriers are added to a pharmaceutically active agent. Eligible are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethylglycols, fatty acid esters of sorbitan. Suspensions according to the invention may use carriers known in the art such as diluents (e.g. water, ethanol or propylene glycol), ethoxylated isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are for example starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid), maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)methylaminol] propane), ethylene diamine, ACES (2-[(amino-2-oxoethyl)amino]ethanesulfonic acid), imidazole, MOPS (3-(N-morphino) propanesulfonic acid), diethyl malonic acid, TES (2-[tris(hydroxymethyl)methyl]aminoethanesulfonic acid), and HEPES (N-2-hydroxyethylpiperazine-N'-2-ethanesulfonic acid).

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), MOPS and N,N-bis-(2-hydroxyethyl)-2-aminoethanesulfonic acid (BES). Another group of preferred buffers are glycine, glycyl-glycine, glycyl-glycyl-glycine, *N,N*-bis-(2-hydroxyethyl)glycine and *N*-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, *N,N,N-*trimethyllysine, 3-methyl histidine, 5-hydroxy-lysine, o-phosphoserine, gamma-carboxyglutamate, [epsilon]-N-acetyl lysine, [omega]-N-methyl arginine, citrulline, ornithine and their derivatives. Particularly preferred is KH₂PO₄ buffer.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorbutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of a sufficient amount of antioxidants is particularly preferable for liquid dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluene, fumaric acid or propyl gallate. Preferred is the use of sodium metabisulfite, alpha-tocopherol and ascorbyl palmitate.

Tablets or pills are usually coated, i.e. the coating constitutes the outer layer. This can be a film coating, a sugar coating with saccharides and a compression coating. Pharmaceutically acceptable varnishes or waxes, HPMC (hydroxypropylmethylcellulose), MC (methylcellulose) or HPC (hydroxypropylcellulose) can be used. Such a coating may help to disguise the taste, to ease the swallowing or the identification. Often plasticizers and pigments are included in the coating. Capsules normally have a gelatinous envelope that encloses the active substance. The specific composition and thickness of this gelatinous layer determines how fast absorption takes place after ingestion of the capsule. Of special interest are sustained release formulations, as known in the art.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising, but not limited to, polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxypropyl starch, hydroxy propyl distarch glycerin, hydroxypropyl distarch phosphate, hydroxypropyl distarch glycerol, starch sodium octenyl succinate, acetylated oxidized starch, hydroxyethyl cellulose.

Suitable pH-regulators for liquid dosage forms are e.g. sodium hydroxide, hydrochloric acid, buffer substances such as sodium dihydrogen phosphate or disodium hydrogen phosphate.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide, magnesium hydroxide, gluconic acid.

Acidifiers use to be inorganic chemicals that either produce or become acid. Suitable examples are: Ammonium chloride, calcium chloride.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum, alginates such as alginic acid and sodium alginate, foaming compositions a.o. Moisture expansion is supported by for example starch, cellulose derivatives, alginates, polysaccharides, dextrans, cross-linked polyvinyl pyrrolidone. The amount of the disintegrant in the composition may vary between 1 and 40% per weight, preferred between 3 and 20% per weight, most preferred between 5 and 10% per weight.

Glidants are materials that prevent a baking of the respective supplements and improve the flow characteristics of granulations so that the flow is smooth and constant. Suitable glidants comprise silicon dioxide, magnesium stearate, sodium stearate, starch and talcum. The amount of the glidant in the composition may vary between 0.01 and 10% per weight, preferred between 0.1 and 7% per weight, more preferred between 0.2 and 5% per weight, most preferred between 0.5 and 2% per weight.

The term lubricants refers to substances that are added to the dosage form in order to facilitate tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15% per weight, preferred between 0.2 and 5% per weight, more preferred between 0.3 and 3% per weight, most preferred between 0.3 and 1.5% per weight. Suitable lubricants are a.o. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Suitable as surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates.

Stabilizers are substances that can be added to prevent unwanted changes. Though stabilizers are not real emulsifiers they may also contribute to the stability of emulsions. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs in order to handle minimal amounts of active agents. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthum gum, gum arabic or any combination thereof.

Anti-caking agents (antiadherents) can be added to a supplement or a composition of supplements in order to prevent the formation of lumps and for easing packaging, transport, release from the at least one chamber of the dispensing cap and consumption. Suitable examples include tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethyl siloxane.

Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

In some galenic formulations it may be desirable that a liquid oral dosage form generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some liquid oral dosage forms may appear slightly foamy upon preparation. Though this does not interfere with the desired application it may affect patient compliance in case of a medication. Therefore, it may be desirable to add a pharmaceutically acceptable anti-foaming agent (defoamer). Examples are polydimethylsiloxane, silicone oil or simethicone.

Opacifiers are substances that render the liquid dosage for, opaque, if desired. They must have a refractive index substantially different from the solvent, in most cases here water. At the same time, they should be inert to the other components of the composition. Suitable examples include titanium dioxide, talc, calcium carbonate, behenic acid, cetyl alcohol, or mixtures thereof.

Suitable fatliquors are e.g. oleic acid decyl ester, hydrated castor oil, light mineral oil, mineral oil, polyethylene glycol, sodium laurylsulfate.

Consistency enhancers are e.g. cetyl alcohol, cetyl ester wax, hydrated castor oil, microcrystalline waxes, non-ionic emulsifier waxes, beeswax, paraffin or stearyl alcohol.

Suitable hydrotropes are alcohols such as ethanol, isopropyl alcohol or polyols such as glycerin.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from achillea, sage, cedar, clove, chamomile, anise, aniseed, star anise, thyme, tea tree, peppermint, mint oil, menthol, cineol, borneol, zingerol, eucalyptus, mango, figs, lavender oil, chamomile blossoms, pine needle, cypress, orange, rose, rosewood, plum, currant, cherry, birch leaves, cinnamon, lime, grapefruit, tangerine, juniper, valerian, lemon, lemon balm, lemon grass, palmarosa, cranberry, pomegranate, rosemary, ginger, pineapple, guava, echinacea, ivy leave extract, blueberry, kaki, melon, alpha- or beta-pinene, alpha-pinene oxide, alpha-campholenic aldehyde, alpha-citronellol, alpha-isoamyl-cinnamic, alpha-cinnamic terpinene, alpha-terpineol, alpha-terpinene, aldehyde C₁₆, alpha-phellandrene, amyl cinnamic aldehyde, amyl salicylate, anisic aldehyde, basil, anethole, bay, benzyl acetate, benzyl alcohol, bergamont, bitter orange peel, black pepper, calamus, camphor, cananga oil, cardamom, carnation, carvacrol, carveol, cassia, castor, cedarwood, cinnamaldehyde, cinnamic alcohol, cis-pinane, citral, citronella, citronellal, citronellol dextro, citronellol, citronellyl acetate; citronellyl nitrile, citrus unshiu, clary sage, clove bud, coriander, corn, cotton seed, d-dihydrocarvone, decyl aldehyde, diethyl phthalate, dihydroanethole, dihydrocarveol, dihydrolinalool, dihydromyrcene, dihydromyrcenol, dihydromyrcenyl acetate; dihydroterpineol, dimethyl salicylate, dimethyloctanal, dimethyloctanol, dimethyloctanyl acetate, diphenyl oxide, dipropylene glycol, d-limonen, d-pulegone, estragole, ethyl vanillin, eucalyptol; eucalyptus citriodora, eucalyptus globulus, eugenol, evening primrose, fenchol, fennel, ferniol, fish, florazon, galaxolide, geraniol, geranium, geranyl acetate, geranyl nitrile, guaiacol, guaiacwood, gurjun balsam, heliotropin, herbanate, hiba, hydroxycitronellal, i-carvone, i-methyl acetate, ionone, isobutyl quinoleine, isobornyl acetate, isobornyl methylether, isoeugenol, isolongifolene, jasmine, lavender, limonen, linallol oxide, linallol, linalool, linalyl acetate, linseed, litsea cubeba, I-methyl acetate, longifolene, mandarin, mentha, menthane hydroperoxide, menthol crystals, menthol laevo, menthone laevo, methyl anthranilate, methyl cedryl ketone, methyl chavicol, methyl hexyl ether, methyl ionone, methyl salicylate, mineral, mint, musk ambrette, musk ketone, musk xylol, myrcene, nerol, neryl acetate, nonyl aldehyde, nutmeg, orris root, para-cymene, parahydroxy phenyl butanone crystals, patchouli, p-cymene, pennyroyal oil, pepper, perillaldehyde, petitgrain, phenyl ethyl alcohol, phenyl ethyl propionate, phenyl ethyl-2methylbutyrate, pimento berry, pimento leaf, pinane hydroperoxide, pinanol, pine ester, pine, pinene, piperonal, piperonyl acetate, piperonyl alcohol, plinol, plinyl acetate, pseudo ionone, rhodinol, rhodinyl acetate, rosalin, ryu, sandalwood, sandenol, sassafras, sesame, soybean, spearmint, spice, spike lavender, spirantol, starflower, tea seed, terpenoid, terpineol, terpinolene, terpinyl acetate, tert-butylcyclohexyl acetate, tetrahydrolinalool, tetrahydrolinalyl acetate, tetrahydromyrcenol, thulasi, thymol, tomato, trans-2-hexenol, trans-anethole, turmeric, turpentine, vanillin, vetiver, vitalizair, white cedar, white grapefruit, wintergreen etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

According to the invention all of the aforementioned excipients and classes of excipients can be used without limitation alone or in any conceivable combination thereof, as long as the inventive use is not thwarted, toxic actions may occur, or respective national legislations are infracted.

5-amino-2,3-dihydro-1,4-phthalazinedione or one of its pharmaceutically acceptable salts can be used as monotherapy or can further be combined with at least one further active ingredient selected from a group comprising active ingredients used in disease-modifying therapies for acute lung injury, in symptomatic therapies of acute lung injury and in the treatment of comorbidities.

Comorbidities can result from impairments due to acute lung injury or are independent thereof. Thus, 5-amino-2,3-dihydro-1,4-phthalazinedione or one of its pharmaceutically acceptable salts can be combined with at least one further active ingredient selected from a group comprising steroidal and non-steroidal anti-inflammatory drugs; immunomodulators; immunosuppressive agents; anti-infective agents like antibiotics, antiretroviral agents, antiviral agents, antifungal agents and antiprotozoal agents; analgesics; anticoagulants; antiplatelet drugs; bronchodilators; pulmonary vasodilators; mucolytic agents; pulmonary surfactants; antioxidants; ENaC-activating agents; HMG-CoA reductase inhibitors, calcium antagonists or AT₁ receptor antagonists for use in the prophylaxis or treatment of acute lung injury.

Suitable examples for such steroidal anti-inflammatory drugs comprise corticosteroids, glucocorticoids, cortisone, cortisone acetate, hydrocortisone, hydrocortisone acetate, dexamethasone, betamethasone, prednisone, prednisolone, methylprednisolone, deltasone, triamcinolone, tixocortol pivalate, mometasone, amcinonide, budesonide, desonide, fluociconide, fluocinolone, halcinonide, fluocortolone, hydrocortisone-17-valerate, halometasone, alclometasone dipropionate, betamethasone valerate, betamethasone dipropionate, prednicarbate, clobetasone-17-butyrate, clobetasol-17-propionate, fluocortolone caproate, fluocortolone pivalate, fluprednidene acetate, hydrocortisone-17-butyrate, hydrocortisone-17-aceponate, hydrocortisone-17-buteprate, ciclesonide, flunisolide, fluticasone furoate, fluticasone propionate, triamcinolone acetonide, beclomethasone dipropionate.

Suitable examples for such non-steroidal anti-inflammatory drugs (NSAIDs) comprise acetylsalicylic acid, salicylic acid and salicylates, acetaminophen (paracetamol), salsalate, diflunisal, ibuprofen, dexibuprofen, naproxen, fenoprofen, ketoprofen, dexketoprofen, flurbiprofen, oxaprozin, loxoprofen, indomethacin, tolmetin, sulindac, etodolac, ketorolac, diclofenac, aceclofenac, nabumetone, piroxicam, meloxicam, tenoxicam, droxicam, lornoxicam, isoxicam, phenylbutazone, mefenamic acid, meclofenamic acid, flufenamic acid, tolfenamic acid, celexoxib, rofecoxib, valdecoxib, parecoxib, lumiracoxib, etoricoxib, firocoxib, nimesulide, clonixin, licofelone, H-harpagide, flunixin, tiaprofenic acid.

Suitable examples for such immunomodulators amongst others comprise thalidomide, lenalidomide, pomalidomide and apremilast.

Suitable examples for such immunosuppressive drugs comprise the groups of glucocorticoids such as listed above; cytostatic drugs such as alkylating agents (such as cyclophosphamide), antimetabolites such as methotrexate, azathioprine, mercaptopurine, fluorouracil, leflunomide, protein synthesis inhibitors and certain antibiotics such as dactinomycin, anthracyclines, mitomycin C, bleomycin and mithramycin, intercalating agents such as mitoxantrone; antibodies such as muromonab-CD3, rituximab, ustekinumab, alemtuzumab, natalizumab, basiliximab and daclizumab; drugs acting on immunophilins such as ciclosporin, tacrolimus and sirolimus, non-classified immunosuppressive agents such as beta-interferon and gamma-interferon, opioids, TNF binding proteins such as infliximab, etanercept, adalimumab; or curcumin, catechins, mycophenolic acid, fingolimod, myriocin and fumaric acid dimethyl esters.

Anti-infective agents is a generic term for compounds that are useful in the treatment of bacterial, viral, fungal, and parasite infections (e.g. protozoa or worms) and comprises antibiotics, antiviral agents, antimycotics agents and antiprotozoal agents.

Suitable examples for such antibiotics comprise imipenem, meropenem, ertapenem, cephalosporins, aztreonam, penicillins such as penicillin G and penicillin V, piperacillin, mezlocillin, ampicillin, amoxicillin, flucloxacillin, methicillin, oxacillin, clavulanic acid, sulbactam, tazobactam, sultamicillin, fosfomycin, teicoplanin, vancomycin, bacitracin, colistin, gramicidin, polymyxin B, tyrothricin, teixobactin, fosmidomycin, amikacin, gentamicin, kanamycin, neomycin, netilmicin, streptomycin, tobramycin, chloramphenicol, fusidic acid, cethromycin, narbomycin, telithromycin, clindamycin, lincomycin, daptomycin, dalfopristin, quinupristin, azithromycin, clarithromycin, erythromycin, roxithromycin, linezolid, doxycycline, minocycline, tetracycline, oxytetracycline, tigecycline, norfloxacin, enoxacin, ciprofloxacin, ofloxacin, levofloxacin, moxifloxacin, metronidazole, tinidazole, aminocumarine, sulfadiazine, sulfadoxin, sulfamethoxazole, sulfasalazine, pyrimethamine, trimethoprim, rifampicin.

The following antiviral agents from HIV, respectively anti-retroviral therapy may be suitable for combination therapy:
Reverse transcriptase inhibitors suitable for such a combination therapy are nucleoside reverse transcriptase inhibitors (NRTI) and non-nucleoside reverse transcriptase inhibitors (NNRTI). Examples of NRTI include, but are not limited to, abacavir, didanosine, emtricitabine, lamivudine, stavudine, tenofovir, zidovudine, zalcitabine, entecavir, adefovir, elvucitabine, fosalvudine(-tidoxil), fozivudintidoxil, lagiciclovir, alamifovir, clevudine, pradefovir, telbivudine. Examples of NNRTI include, but are not limited to, efavirenz, etravirine, nevirapine, rilpivirine, delavirdine, emivirine, lersivirine.

Suitable for a combination therapy according to the invention are integrase inhibitors such as raltegravir, elvitegravir, dolutegravir, MK-2048.

Examples of HIV protease inhibitors suitable for a combination therapy according to the invention are saquinavir, indinavir, ritonavir, nelfinavir, amprenavir, lopinavir, atazanavir, fosamprenavir, tipranavir, darunavir, brecanavir, mozenavir, tipranavir.

Examples of entry inhibitors suitable for a combination therapy according to the invention are enfuvirtide and maraviroc.

Suitable examples for general antiviral drugs comprise ancriviroc, aplaviroc, cenicriviroc, enfuvirtide, maraviroc, vicriviroc, amantadine, rimantadine, pleconaril, idoxuridine, aciclovir, brivudine, famciclovir, penciclovir, sorivudine, valaciclovir, cidofovir, ganciclovir, valganciclovir, sofosbusvir, foscarnet, ribavirine, taribavirine, filibuvir, nesbuvir, tegobuvir, fosdevirine, favipiravir, merimepodib, asunaprevir, balapiravir, boceprivir, ciluprevir, danoprevir, daclatasvir, narlaprevir, telaprevir, simeprevir, vanipevir, rupintrivir, fomivirsen, amenamevir, alisporivir, bevirimate, letermovir, laninamavir, oseltamivir, peramivir, zanamivir, remdesivir.

Suitable examples for such antimycotic drugs comprise abafungin, amphotericin B, candicidin, filipin, hamycin, natamycin, nystatin, rimocidin, bifonazole, butoconazole, clotrimazole, econazole, fenticonazole, isoconazole, ketoconazole, luliconazole, miconazole, omoconazole, oxiconazole, sertaconazole, sulconazole, tioconazole, albaconazole, efinaconazole, epoxiconazole, fluconazole, isavuconazole, itraconazole, posaconazole, propiconazole, ravuconazole, terconazole, voriconazole, amorolfin, butenafine, nafitifine, terbinafine, anidulafungin, caspofungin, micafungin, benzoic acid, ciclopirox, flucytosine, griseofulvin, haloprogin, tolnaftate, undecylenic acid, crystal violet, balsam of Peru.

Suitable examples for such antiprotozoal drugs comprise metronidazole, tinidazole, ornidazole, atovaquone, clioquinol, chlorquinaldol, emetin, pentamidine isethionate, eflornithine, nitrofural, halofuginone, miltefosine, chloroquine, hydroxychloroquine, mepacrine, primaquine, amodiaquine, pamaquine, piperaquine, proguanil, cyclohunailembonate, quinine, mefloquine, pyrimethamine, artmether, artemisinin, artesunate, dihydroartemisinine, halofantrine, lumefantrine, sulfadoxine.

Suitable examples for further antiparasitic drugs comprise meglumine antimoniate, benznidazole, sodium stibogluconate, fumagillin, halofantrine, melarsoprol, nifurtimox, nitazoxanide, permethrin, lindane, malathion, carbaryl, pyrethrum, phenothrin, bio-allethrin, imidacloprid, moxidectin, nitenpyram, fipronil, pyriprol, selamectin, dimpylate, spinosad, indoxacarb, methoprene, pyriproxyfen, lufenuron, neem oil, citronella oil, clove oil, peppermint oil, eucalyptus oil.

Suitable examples for analgesics comprise the NSAIDs listed above; opioid analgesics such as morphine, fentanyl, methadone, oxycodon, carfentanyl, dihydroetorphin, ohmefentanyl, etorphin, sufentanil, remifentanil, alfentanil, buprenorphine, hydromorphone, levomethadone, hydrocodone, pintramide, nalbuphine, tapentadol, pentazocin, dihydrocodeine, codeine, pethidine, tramadol, tilidine, meptazinol, naloxone, naltrexone, diprenorphine, loperamide, apomorphine; epibatidine; scopolamine; ziconitide; cannabinoids such as tetrahydrocannabinol, cannabidiol, marinol; flupirtine; ketamine and local anesthetics listed above.

Suitable examples for such anticoagulants comprise heparins, coumarins such as phenprocoumon (marcumar) and warfarin, apixaban, rivaroxaban, edoxaban, dabigatran, ximelagatran, hirudine, lepirudine, bivalirudine, citrate, EDTA, fondaparinux, argatroban, otamixaban.

Suitable examples for such antiplatelet agents comprise abciximab, acetylsalicylic acid, dipyridamole, clopidogrel, eptifibatide, ilomedin, prostacyclin, prasugrel, ticagrelor, ticlopidine and tirofiban.

Suitable bronchodilators such as beta-2 adrenergic receptor agonists comprise short-acting beta-2 agonists (SABAs) such as salbutamol, albuterol, bitolterol, fenoterol, isoprenaline, levosalbutamol, levalbuterol, orciprenaline, pirbuterol, procaterol, ritodrine and terbutaline; long-acting beta-2 agonists (LABAs) such as arformoterol, bambuterol, clenbuterol, formoterol and salmeterol; ultra-long-acting beta-2 agonists such as abediterol, carmoterol, indacaterol, olodaterol and vilanterol, alone or combined with umeclidinium bromide and/or fluticasone furoate; beta-2 agonists with unknown time of action such as isoxsuprine, mabuterol or zilpaterol.

Suitable muscarinic anticholinergics (bronchodilating M₃ receptor antagonists) comprise ipratropium bromide, tiotropium bromide, oxitropium bromide, glycopyrronium bromide, aclidinium bromide, umeclidinium bromide, atropine, hyoscyamine, aclidinium bromide, 4-DAMP, darifenacin, DAU-5884, HL-031, HL-120, J-104, J-129, procyclidine, oxybutynin, tolterodine and zamifenacin.

Further bronchodilators comprise epinephrine, ephedrine, theophylline and TSG12.

A potent pulmonary vasodilator is nitric oxide. Further suitable pulmonary vasodilators are prostacyclin (prostaglandin PGI₂) analogues such as iloprost, epoprostenol and treprostinil.

Suitable mucolytic agents comprise N-acetylcysteine (NAC), ambroxol, bromhexine, carbocisteine, erdosteine, mecysteine and dornase alfa.

Suitable pulmonary surfactants comprise synthetic compositions such as Colfosceril palmitate, Pumactant, KL-4, Venticute and Lucinactant as well as animal-derived surfactants such as Beractant, Calfactant and Poractant alfa.

A potent antioxidant is inhaled carbon monoxide (CO).

Suitable ENaC (epithelium sodium channel) activating peptides comprise AP301 and S3969.

Suitable HMG-CoA reductase inhibitors (statins) comprise atorvastatin, alone or in combination with amlodipine and/or perindopril, cerivastatin, fluvastatin, lovastatin, alone or in combination with niacin, mevastatin, pitavastatin, pravastatin, rosuvastatin, alone or in combination with ezetimibe, simvastatin, alone or in combination with ezetimibe or niacin.

Suitable calcium antagonists comprise verapamil, gallopamil, fendiline, nimodipine, nifedipine, nitrendipine, amlodipine, felodipine, lercanidipine, nicardipine, lacidipine, isradipine, nisoldipine, nivaldipine, manidipine, clevidipine, aranidipine, azelnidipine, barnidipine, benidipine, cilnidipine, efonidipine, pranidipine, diltiazem, mibefradil, bepridil, flunarizine and fluspiriline.

Suitable AT₁ antagonists (angiotensin II receptor blockers; sartans) comprise losartan, valsartan, candesartan, telmisartan, irbesatan, olmesartan, eprosartan, fimasartan, azilsartan, milfasartan, pomisartan, pratosartan, ripisartan, tasosartan, saprosartan and EXP 3174.

5-amino-2,3-dihydro-1,4-phthalazinedione or one of its pharmaceutically acceptable salts and the further active ingredient can be used simultaneously, separately or sequentially in order to treat or prevent disease symptoms. The two active agents may be provided in a single dosage form or as separate formulation, each formulation containing at least one of the two active agents. One or both of the two active agents may be formulated as a bolus.

In particular, the present application discloses 5-amino-2,3-dihydro-1,4-phthalazinedione or one of its pharmaceutically acceptable salts, a composition according to the invention or a combination according to the invention for use in the treatment of acute lung injury, wherein a previous treatment with at least one other pharmaceutically active agent was refractory.

Pharmaceutical formulations suitable for oral dosage forms for 5-amino-2,3-dihydro-1,4-phthalazinedione or one of its pharmaceutically acceptable salts, a composition according to the invention or a combination according to the invention may be administered as separate units such as soft gelatin capsules, hard gelatin capsules, tablets, sugar-coated tablets or pills; powders or granulates; juices, syrups, drops, teas, solutions or suspensions in aqueous or non-aqueous liquids; edible foams or mousses; or in oil-in-water or water-in-oil in emulsions.

In oral dosage forms such as tablets or capsules the active agent can thus be combined with a non-toxic and pharmaceutically acceptable inert carrier such as ethanol, glycerol or water. Powders are produced by grinding the compound to a suitably tiny particle size and mixing them with a pharmaceutical carrier in a similar manner, e.g. an edible carbohydrate such as starch or mannitol. A flavor, preservative, dispersant or colorant can also be present.

Tablets are formulated by producing, granulating or dry-pressing a powder mixture, adding a lubricant and a disintegrants and pressing the mixture to a tablet. A powder mixture is produced by mixing a suitably ground compound with a diluent or a base as described before, and if applicable, with a binding agent such as carboxymethyl cellulose, an alginate, gelatin or polyvinyl pyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acacia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting mold. The lubricated mixture is then pressed to give tablets. The compounds according to the invention can also be combined with a free-flowing inert excipient and then pressed directly to give tablets without carrying out the granulation or dry-pressing steps.

In another aspect of the invention 5-amino-2,3-dihydro-1,4-phthalazinedione or one of its pharmaceutically acceptable salts is provided in hard gelatin capsules. They are fabricated by producing a powder mixture as described before and filling it into shaped gelatin covers. Glidants and lubricants such as highly dispersed silica, talcum, magnesium stearate, calcium stearate or polyethylene glycol can be added to the powder mixture as solids. A disintegrant or solubilizer such as agar agar, calcium carbonate or sodium carbonate can be added likewise in order to improve the availability of the medication after intake of the capsule. Additionally, suitable binding agents and/or colorants can be added to the mixture, if desirable or necessary.

In another aspect of the invention 5-amino-2,3-dihydro-1,4-phthalazinedione or one of its pharmaceutically acceptable salts is included in soft gelatin capsules (SGC). SGCs are dissolved on their passage through the gastrointestinal tract. They consist mainly of gelatin enriched with variable amounts of plasticizers such as glycerol or sorbitan. The release rate depends on the specific formulation of the SGC carrier material. They are also suitable for a sustained release of the active agent. SGCs are particularly useful for the administration of poorly water-soluble active agents.

In another aspect of the invention 5-amino-2,3-dihydro-1,4-phthalazinedione or one of its pharmaceutically acceptable salts is included in chewable tablets or hard caramels. Herein the substance is integrated into the matrix of the tablets or caramels.

Sublingual drug delivery can be an alternative when compared to oral drug delivery as sublingually administered dosage forms bypass hepatic metabolism. A rapid onset of pharmacological effect is often desired for some drugs, especially those used in the treatment of acute disorders. Sublingual tablets disintegrate rapidly, and the small amount of saliva present is usually sufficient for achieving disintegration of the dosage form coupled with better dissolution and increased bioavailability.

The drug must be lipophilic enough to be able to partition through the lipid bilayer, but not so lipophilic such that once it is in the lipid bilayer, it will not partition out again. According to the diffusive model of absorption, the flux across the lipid bilayer is directly proportional to the concentration gradient. Therefore, lower salivary solubility results in lower absorption rates and vice versa. In general, a drug which has been formulated for sublingual should ideally have a molecular weight of less than 500 to facilitate its diffusion. The oral cavity has a narrow pH range which lies between 5.0 to 7.0. The inclusion of a suitable buffer during the formulation of an ionizable drug makes it possible to control the pH of aqueous saliva.

In order to avoid a possibly unpleasant taste or smell of the drug taste masking is needed. Sweeteners, flavors, and other taste-masking agents are essential components. Sugar-based excipients quickly dissolve in saliva and produce endothermic heat of dissolution. They create a pleasant feeling in the mouth and are most suitable for sublingual tablets along with other flavors.

Typical techniques for manufacturing sublingual tablets include direct compression, compression molding, freeze drying and hot melt extrusion (Khan et al. (2017) J Pharmaceut Res 16: 257-267).

When swallowing is avoided, an administration of a pharmaceutically active agent by means of a sublingual tablet can also reach the pharynx/throat topically. Absorption of the pharmaceutically active agent occurs to a good part via the pharyngeal mucosa.

A lozenge (troche) is a small, disc-shaped or rhombic body composed of solidifying paste containing an astringent, antiseptic, or demulcent drug, used for local treatment of the mouth or throat, the lozenge being held in the mouth until dissolved. The vehicle or base of the loze nge is usually sugar, made adhesive by admixture with acacia or tragacanth, fruit paste, made from black or red currants, confection of rose, or balsam of tolu.

The present application also discloses the parenteral administration of 5-amino-2,3-dihydro-1,4-phthalazinedione or one of its pharmaceutically acceptable salts, a composition according to the invention or a combination according to the invention in the prophylaxis or treatment of acute lung injury in the form of intravenous injection, intraarterial injection or intraperitoneal injection.

These liquid dosage forms comprise solutions, suspensions and emulsions. Examples are water and water/propylene glycol solutions for parenteral injections, or the addition of a sweetener or opacifier for oral solutions, suspensions and emulsions.

These liquid dosage forms can be stored in vials, IV bags, ampoules, cartridges, and prefilled syringes. Suitable excipients include solubilizers, stabilizers, buffers, tonicity modifiers, bulking agents, viscosity enhancers/reducers, surfactants, chelating agents, and adjuvants.

In yet another aspect of the invention the present application discloses 5-amino-2,3-dihydro-1,4-phthalazinedione or one of its pharmaceutically acceptable salts, a composition according to the invention or a combination according to the invention for use in the prophylaxis or treatment of acute lung injury, wherein said substance, composition or combination is formulated as a lyophilizate. A lyophilizate can be reconstituted with water for injection or physiological saline or a water/ethanol solution and then be administered by injection.

Typical application forms for intravenous injections include infusion pumps, hypodermic needles, drip chambers, peripheral cannulas (peripheral venous catheters) and pressure bags.

In yet another aspect of the invention 5-amino-2,3-dihydro-1,4-phthalazinedione or one of its pharmaceutically acceptable salts, a composition according to the invention or a combination according to the invention is provided as a formulation for inhalation.

For an effective prophylactic or therapeutic treatment of acute lung injury it is advantageous that 5-amino-2,3-dihydro-1,4-phthalazinedione or one of its pharmaceutically acceptable salts, a composition according to the invention or a combination according to the invention reaches the patient's lower airways, in particular the alveoli. Therefore, the particle size must be sufficiently small to reach the lowest parts of the airways of the pulmonary tissue. The best inhalatory device class for inhalatory application of a pharmaceutically active agent are the so-called mesh nebulizers described before. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable commercially available mesh nebulizers, jet nebulizers, ultrasonic nebulizers, dry powder inhalers and (pressurized) metered-dose inhalers comprise, without being limiting, PARI eFlow^{®}rapid, PARI LC STAR^{®}, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB^{®}-ir, OPTI-NEB^{®}, M-neb^{®} dose⁺ mesh nebulizer inhalation MN-300/8, M-Neb Flow+ and M-neb^{®} mesh nebulizer MN-300/X (NEBU-TEC, Eisenfeld, Germany), Hcmed Deepro HCM-86C and HCM860 (HCmed Innovations Co., Ltd, Taipei, Taiwan), OMRON MicroAir U22 and U100 (OMRON, Kyoto, Japan), Aerogen^{®} Solo, Aerogen^{®} Ultra and Aerogen^{®} PRO (Aerogen, Galway, Ireland), KTMED NePlus NE-SM1 (KTMED Inc., Seoul, South Korea), Vectura Bayer Breelib^{™} (Bayer AG, Leverkusen, Germany), Vectura Fox, MPV Truma and MicroDrop^{®} Smarty (MPV MEDICAL GmbH, Kirchheim, Germany), MOBI MESH (APEX Medical, New Taipei City, Taiwan), B.Well WN-114, TH-134 and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Bia 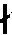 ystok, Poland), DIGI O₂ (DigiO₂ International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump^{®} KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist^{®} (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus^{®} BBU01 and BBU02 (Tai Yu International Manufactory Ltd., Dongguan, China), TaiDoc TD-7001 (TaiDoc Technology Co., New Taipei City, Taiwan), Vibralung^{®} and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed^{®} JLN-MB001 (Kernmed, Durmersheim, Germany).

Preferred are mesh nebulizers with a piezoelectric activation of the nebulization process, respectively vibrating mesh nebulizers.

Thus, in another aspect of the invention the present application relates to 5-amino-2,3-dihydro-1,4-phthalazinedione or one of its pharmaceutically acceptable salts, a composition according to the invention or a combination according to the invention for use in the prophylaxis or treatment of acute lung injury in a formulation for inhalatory administration, wherein the inhalatory administration is carried out by means of a vibrating mesh nebulizer.

Mesh nebulizers can be classified into two groups according to patient interaction: Continuous mode devices and trigger-activated devices. In continuous mode mesh nebulizers the nebulized aerosol is continuously released into the mouth piece and the patient has to inhale the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, in particular vibrating mesh nebulizers.

Particularly preferred are trigger-activated mesh nebulizers with a piezoelectric activation of the nebulization process.

Preferred are the mesh nebulizer models PARI eFlow^{®}rapid, Philips Respironics I-neb, Philips InnoSpire Go, M-neb^{®} dose⁺ mesh nebulizer inhalation MN-300/8, Hcmed Deepro HCM-86C and HCM860, OMRON MicroAir U100, Aerogen^{®} Solo, KTMED NePlus NE-SM1, Vectura Fox, Vectura Bayer Breelib^{™}.

The most preferred vibrating mesh nebulizer models are high-end models such as PARI eFlow^{®}rapid, PARI Velox, Philips Respironics I-neb, M-neb^{®} dose⁺ mesh nebulizer inhalation MN-300/8, Aerogen^{®} Solo, Vectura Fox, Vectura Bayer Breelib^{™}.

Thus, the present application refers to 5-amino-2,3-dihydro-1,4-phthalazinedione or one of its pharmaceutically acceptable salts, a composition according to the invention or a combination according to the invention for use in the prophylaxis or treatment of acute lung injury, wherein said substance, composition or combination is applied by inhalation by using a vibrant mesh nebulizer, metered dose-inhaler or dry-powder inhaler.

The mean droplet size is usually characterized as MMAD (median mass aerodynamic diameter). The individual droplet size is referred to as MAD (mass aerodynamic diameter). This value indicates the diameter of the nebulized particles (droplets) at which 50% are smaller or larger, respectively. Particles with a MMAD > 10 µm normally do not reach the lower airways, they often get stuck in the throat. Particles with a MMAD > 5 µm and < 10 µm usually reach the bronchi but not the alveoli. Particles between 100 nm and 1 µm MMAD don't deposit in the alveoli and are exhaled immediately. Therefore, the optimal range is between 1 µm and 5 µm MMAD. Recent publications even favor a narrower range between 3.0 µm and 4.0 µm (cf. Amirav et al. (2010) J Allergy Clin Immunol 25: 1206-1211; Haidl et al. (2012) Pneumologie 66: 356-360).

A further commonly accepted quality parameter is the percentage of the particles in the generated aerosol with a diameter in the range of 1 µm to 5 µm (FPM; fine particle mass). FPM is a measure for the particle distribution. It is calculated by subtracting the percentage of the particles in the generated aerosol with a diameter in the range < 1 µm from the overall percentage of the particles in the generated aerosol with a diameter in the range < 5 µm (FPF; fine particle fraction).

In another aspect of the invention the present application refers also to a method for producing an aerosol according to the invention for the prophylaxis or treatment of acute lung injury, comprising the following steps:
a) filling 0.1 ml to 5 ml of an aqueous solution containing 5-amino-2,3-dihydro-1,4-phthalazinedione or one of its pharmaceutically acceptable salts, a composition according to the invention or a combination according to the invention and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

The vibration frequency of vibrating mesh nebulizers is normally in the range of 80 kHz to 200 kHz, preferred 90 kHz to 180 kHz, more preferred 100 kHz to 160 kHz, most preferred 105 kHz to 130 kHz (cf. Chen, The Aerosol Society: DDL2019**;** Gardenshire et al. (2017) A Guide to Aerosol Delivery Devices for Respiratory Therapists, 4th ed.).

Thus, the aforementioned method is also disclosed with said vibration frequency ranges.

The method according to the invention is thus characterized in that at least 80 % in weight, preferred at least 85 % in weight, most preferred at least 90 % in weight of 5-amino-2,3-dihydro-1,4-phthalazinedione or one of its pharmaceutically acceptable salts, a composition according to the invention or a combination according to the invention contained in said aqueous solution are nebulized in the generated aerosol.

The method of the invention is particularly effective in nebulizing a high percentage of the pharmaceutically active agent(s) from the provided aqueous solution during a short time. This is an important feature for patient compliance. A considerable percentage of the patient population finds the inhalatory process to be uncomfortable, weary and physically demanding. On the other hand, the patient's active cooperation is essential for an effective and targeted inhalatory application. Therefore, it is desirable that a therapeutically sufficient amount is applied during a period of time as short as possible. Surprisingly, it showed that during a three minutes time span 95 % of the substance provided in the aqueous solution could be nebulized. This is an ideal time span for a high patient compliance.

Therefore, the method according to the invention is thus characterized in that at least 80 % of the generated aerosol are produced during three minutes after starting nebulization in the mesh nebulizer, preferred at least 85 % and most preferred at least 90 %.

While the pharmaceutically active agent is usually provided in a single dosage container for every nebulization procedure the nebulizer and/or the mouthpiece can be used over a certain period of time and have to be replaced at certain intervals. A cleaning of the nebulizer and the mouthpiece is recommended by default after each nebulization. But herein patient compliance cannot be reasonably taken for granted. But even after a meticulous cleaning there are always some deposits of the aerosol in the nebulization chamber, the outlet and/or the mouthpiece. As the aerosol is produced from an aqueous solution these depositions bear the risk of producing a bioburden of bacteria that might contaminate the inhaled aerosol. Deposits may also plug holes in the mesh membrane of the mesh nebulizer. In general, the nebulizer and/or the mouthpiece should be exchanged every one or two weeks. Therefore, it is convenient to offer the medication and the nebulizer as a combined product.

Vibrating mesh nebulizers delivered better results than ultrasonic or jet nebulizers for administration of antibiotics. When a constant output vibrating-mesh nebulizer is placed on the inspiratory limb at 10 cm of the Y-piece and specific ventilation parameters (tidal volume of 8 ml/kg, respiratory rate of 12 c/min, duty cycle of 50%, constant and low inspiratory flow rate inferior to 30 I/min and end inspiratory pause of 20%) are set, 63% of the administered drug (ceftazidime, amikacin) reach the inlet of the endotracheal tube, versus 37% extrapulmonary deposition (Lu et al. (2011) Am J Respir Crit Care Med 184: 106-115). Mostly, the administered drug is evenly distributed between both lungs. In pigs, the use of helium (He/O₂) instead of nitrogen (N₂/O₂) in inhaled gas was found to increase ceftazidime concentrations in subpleural lung specimens (Tonnelier et al. (2005) Anesthesiology 102: 995-1000).

In yet another aspect of the invention 5-amino-2,3-dihydro-1,4-phthalazinedione or one of its pharmaceutically acceptable salts, a composition according to the invention or a combination according to the invention for use in the prophylaxis or treatment of acute lung injury, wherein said substance, composition or combination is applied in form of liposomes, micelles, multilamellar vesicles or a cyclodextrin complex. A method for producing multilamellar vesicles of 5-amino-2,3-dihydro-1,4-phthalazinedione or its sodium salt is disclosed in WO 2019/137825.

In yet another aspect of the invention 5-amino-2,3-dihydro-1,4-phthalazinedione or one of its pharmaceutically acceptable salts, a composition according to the invention or a combination according to the invention for use in the prophylaxis or treatment of acute lung injury, wherein said substance, composition or combination is provided as an additive to the ventilation air of a cardiopulmonary bypass device, a form of assisted ventilation. When the patient's condition in intensive care unit worsens, they often need to be ventilated in such a device for an indefinite period of time until their own respiration would allow for a sufficient oxygen supply. Good results have been achieved when with an aerosol in a metered-dose inhaler combined with an inhalation chamber at the Y-piece. This can increase the applied dosage of bronchodilators by the factor 1.5 to 4 (Fuller et al. (1994) Chest 105: 214-218). 38% of the pharmaceutically active agent could be delivered (Marik et al. (1999) Chest 115: 1653-1657). Alternatively, a constant output mesh nebulizer yielded rates of 10 - 15%, as assessed in a scintigraphic study (Dugernier et al. (2016) Ann Intensive Care 6: 73). Vibrating mesh nebulizers delivered better results than ultrasonic or jet nebulizers for administration of antibiotics. When a constant output vibrating-mesh nebulizer is placed on the inspiratory limb at 10 cm of the Y-piece and specific ventilation parameters (tidal volume of 8 ml/kg, respiratory rate of 12 c/min, duty cycle of 50%, constant and low inspiratory flow rate inferior to 30 I/min and end inspiratory pause of 20%) are set, 63% of the administered drug (ceftazidime, amikacin) reach the inlet of the endotracheal tube, versus 37% extrapulmonary deposition (Lu et al. (2011) Am J Respir Crit Care Med 184: 106-115). Mostly, the administered drug is evenly distributed between both lungs. In pigs, the use of helium (He/O₂) instead of nitrogen (N₂/O₂) in inhaled gas was found to increase ceftazidime concentrations in subpleural lung specimens (Tonnelier et al. (2005) Anesthesiology 102: 995-1000).

In these cases 5-amino-2,3-dihydro-1,4-phthalazinedione or one of its pharmaceutically acceptable salts can be added to the intubated ventilation air in solid form (dry powder) or in liquid form (in an aqueous solution or as a nebulized aerosol, as described before).

The present application discloses thus also 5-amino-2,3-dihydro-1,4-phthalazinedione or one of its pharmaceutically acceptable salts, a composition according to the invention or a combination according to the invention for use in the prophylaxis or treatment of acute lung injury, wherein said substance, composition or combination is added to the ventilation air of a cardiopulmonary bypass device.

Moreover, a method of treatment of acute lung injury is disclosed, wherein a therapeutically effective amount of 5-amino-2,3-dihydro-1,4-phthalazinedione or one of its pharmaceutically acceptable salts, a composition according to the invention or a combination according to the invention is administered to a patient in need thereof.

### EXAMPLES

In all experiments and treatments, 5-amino-2,3-dihydro-1,4-phthalazinedione sodium salt anhydrous Form I is used (provided by MetrioPharm, synthesized at ChemCon, Freiburg, Germany).

### Example 1: 5-amino-2,3-dihydro-1,4-phthalazinedione sodium salt reduces pro-inflammatory cytokine secretion in murine peritoneal macrophages

Female C57BI/6 mice (6-8 weeks old) were purchased from Envigo (San Petro al Natisone, Udine, Italy) and housed under standard laboratory conditions (specific-pathogen free). Food and water were applied *ad libitum.* Animal handling and the study protocol were in accordance with international guidelines.

Four days after i.p. injection of 3% thioglycolate medium (w/v in distilled water; St Louis, MO USA), murine peritoneal macrophages (PM) were isolated according to the protocol of Zhang et al. (Curr. Protoc. Immunol. 2008, Chapter 14, Unit 14.1).

Briefly, cells were collected by injecting 10 ml of complete medium (DMEM/F12 Medium containing 10% fetal calf serum) into the peritoneal cavity using a 30cc syringe attached to a 19-G needle, followed by slowly withdrawing the lavage fluid. Cells were washed twice in PBS (phosphate buffered saline) and counted. A total of 3-4x10⁶ macrophages were collected from each mouse.

Cell purity was confirmed by flow cytometry (using CD14 expression) and isolated PMs were allowed to adhere overnight. One hour prior to LPS (lipopolysaccharide) stimulation (0.1 µg/ml; *Escherichia coli* O55:B5, St Louis, MO USA), cells (0.5×10⁶ cells/well in 24-well plates with 1 ml total volume of medium) were pre-treated with scalar concentrations of 5-amino-2,3-dihydro-1,4-phthalazinedione sodium salt (ranging from 1 mM to 0.025 mM). Two independent experiments (each using cells pooled from three mice) were performed and secreted cytokine levels were determined 24, 48 and 72 h after LPS stimulation using ELISA technique.

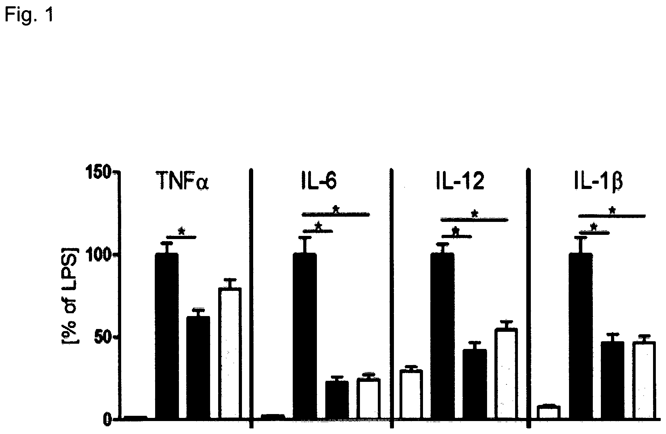

While 5-amino-2,3-dihydro-1,4-phthalazinedione sodium salt did not affect cytokine secretion of unstimulated murine PMs (data not shown), LPS-induced proinflammatory cytokine concentrations were reduced by pre-treatment with 5-amino-2,3-dihydro-1,4-phthalazinedione sodium salt (Fig. 1). In detail, 1 mM of 5-amino-2,3-dihydro-1,4-phthalazinedione sodium salt significantly reduced the levels of TNF-alpha, IL-6, IL-12, and IL-1-beta by about 40%, 80%, 60%, and 50%, respectively. Interestingly, similar effects of 5-amino-2,3-dihydro-1,4-phthalazinedione sodium salt were observed for all cytokines after 48 h and 72 h of culture. Furthermore, these effects were validated in PMs of a second mouse strain (Balb/c) and a clear dose-dependency was observed with six different concentrations of 5-amino-2,3-dihydro-1,4-phthalazinedione sodium salt ranging from 1 mM to 0.25 mM (data not shown).

These experiments show clearly that a treatment with 5-amino-2,3-dihydro-1,4-phthalazinedione sodium salt reduced significantly the secretion of pro-inflammatory cytokines.

### Example 2: 5-amino-2,3-dihydro-1,4-phthalazinedione sodium salt Form I was tested in the PCI model of sepsis

As explained before, severe infections are the most common cause for ALI. They may cause pneumonia or systemic diseases such as sepsis, sepsis syndrome and septic shock (Piantadosi and Schwartz (2004) Ann Intern Med 141: 460-470). Therefore, a sepsis model of infection is also indicative for the efficacy of a pharmaceutically active agent in ALI.

In the PCI (peritoneal contamination and infection) model of sepsis a human fecal suspension was injected intraperitoneally to mice (3 µl/g body weight). To all groups the antibiotic meropenem (25 mg/kg body weight) was administered 6 hours after PCI induction. At this time point all mice received also a volume loading with a 5% glucose solution. This treatment is repeated on the two consecutive days after PCI induction, at intervals of 24 hours.

| | |
|---|---|
| Group I: | control (not infected) |
| Group II: | PCI (infected, without treatment) |
| Group III: | PCI + 2 mg/kg body weight 5-amino-2,3-dihydro-1,4-phthalazinedione sodium salt Form I |
| Group IV: | PCI + 5 mg/kg body weight 5-amino-2,3-dihydro-1,4-phthalazinedione sodium salt Form I |

### a) Therapeutic mode

Starting at 8 hours after PCI induction the mice of the treatment groups were treated for two consecutive days every 8 hours with an intraperitoneal injection of 2 mg/kg body weight, respectively 5 mg/kg body weight 5-amino-2,3-dihydro-1,4-phthalazinedione sodium salt Form I. Thereupon the mice were treated for further eight days with an intraperitoneal injection of 2 mg/kg body weight, respectively 5 mg/kg body weight 5-amino-2,3-dihydro-1,4-phthalazinedione sodium salt Form I once daily. (n = 6)

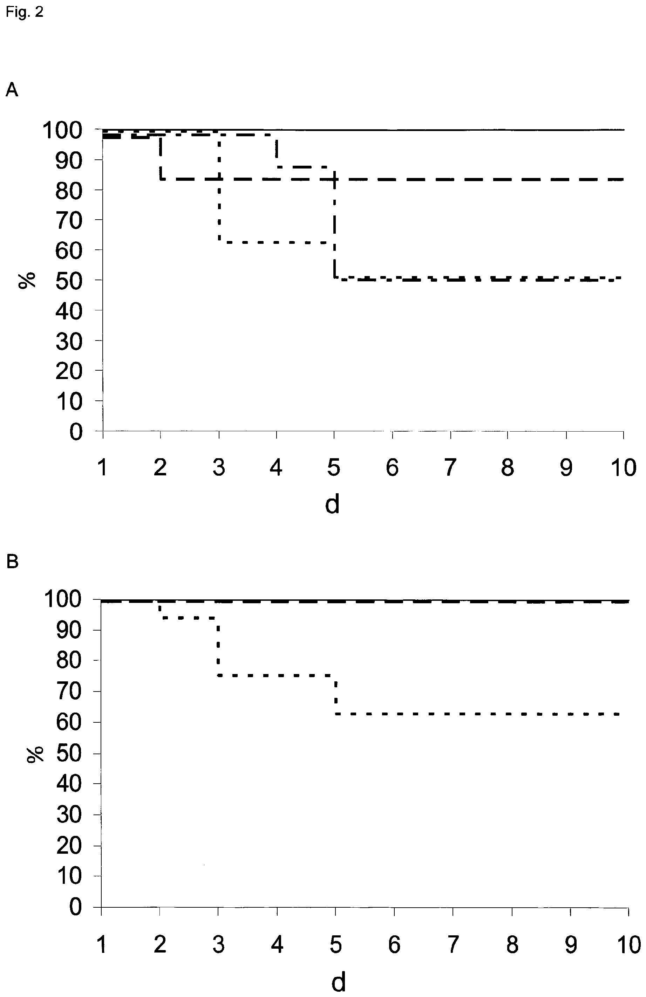

The mortality of the mice was assessed daily. As expected, all mice of the uninfected group (Group I) survived. In Group II two mice died on Day 3 and one mouse on Day 5. Thus, there is a survival rate of 50% after 10 days. In Group III one mouse died on Day 4 and two mice died on Day 5. Thus, there is also a survival rate of 50% after 10 days. In Group IV one mouse died on Day 2 and all other mice survived. Thus, there is a survival rate of 83% after 10 days. It can be seen that the daily treatment with a dose of 5 mg/kg body weight 5-amino-2,3-dihydro-1,4-phthalazinedione sodium salt Form I clearly reduces the mortality of mice in the PCI model. The graphic evaluation is shown in Fig. 2A.

### b) Prophylactic mode

Herein the mice were treated with 5 mg/kg body weight 5-amino-2,3-dihydro-1,4-phthalazinedione sodium salt Form I only, but only twice, 3 hours before PCI induction and 6 hours after (n = 8). All mice of Group I survived. In Group II one mouse died on Day 2, another on Day 3 and yet another on Day 5. Thus, there is a survival rate of 63%. In Group IV none of the mice died. Thus, there is a survival rate of 100%. This shows that a prophylactic administration of 5-amino-2,3-dihydro-1,4-phthalazinedione sodium salt can completely prevent the mortality of mice in the PCI model of sepsis. The graphic evaluation is shown in Fig. 2B.

### c) Clinical Severity Score

The Clinical Severity Score (CSS) was assessed daily for the mice of the prophylactic treatment group. The CSS was assessed according to Gonnert et al. (2011) J Surg Res 170: e123-134.

| **Grade** | **Quality** | **Spontaneous activity** | **Reaction to exogenous stimuli** | **Posture** |
|---|---|---|---|---|
| 1 | no signs of illness | active, strong | curious, quick movements | normal |
| 2 | low-grade | less active with occasional interruptions in activity | reduced alertness, but adequate response | slightly hunched |
| 3 | mid-grade | slow, sleepy, moves with difficulty | limited and delayed | hunched |
| 4 | high-grade | lethargic, motionless, no movement | none | severely hunched |

In comparison to the untreated group the surviving animals of the prophylactically treated group (5 mg/kg body weight 5-amino-2,3-dihydro-1,4-phthalazinedione sodium salt Form I) were less strongly diseased and they also recovered clearly more quickly, in particular after Day 4. This is depicted in Fig. 2C.

### Example 3: 5-amino-2,3-dihydro-1,4-phthalazinedione sodium salt Form I was tested in an isolated, ventilated and perfused mouse lung system that was stimulated with cigarette smoke.

The isolated, ventilated and perfused mouse lung system (ILU) is an established model for studying acute effects of various conditions and drugs on lung parenchyma and vasculature. It is mainly used for examining effects of hypoxia and for evaluating the efficacy of potential drugs on the hypoxic pulmonary vasoreaction (cf. Weissmann et al. (2006) Proc Natl Acad Sci USA 103: 19093-19098). Results from this experimental set-up are regarded as not only indicative for the treatment of COPD but to all inflammatory disorders of the lower airways.

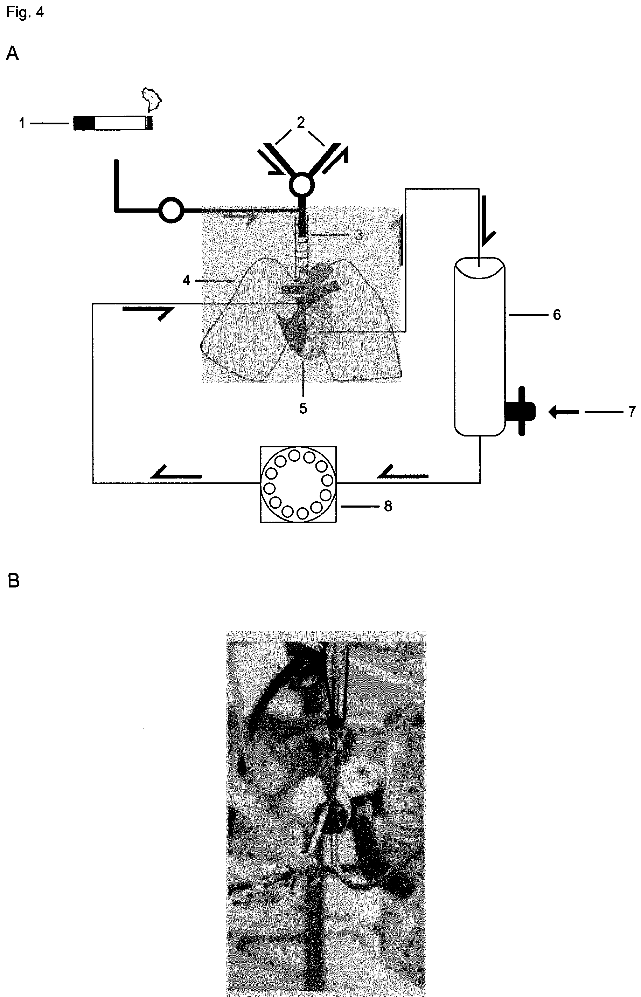

C57BL/6J mice (n = 25, 5 per group; male/female, 3 - 6 months, 20 - 30 g; Charles River GmbH, Sulzfeld, Germany) were anesthetized with a ketamine (100 mg/kg body weight) and xylazine (20 mg/kg body weight) intraperitoneal injection (Ceva Tiergesundheit GmbH, Düsseldorf, Germany) containing heparin (50 I.E. heparin/g body weight; Ratiopharm GmbH, Ulm, Germany). The lungs and the heart were removed from the chest cavity and placed on the ILU system (see Fig. 1A and 1B). Lungs were ventilated in an isolated chamber using normoxic gas (21% O₂, 5% CO₂, 74% N₂; 150 breaths per minute at the PEEP (positive end-expiratory pressure) of 3 cm H₂O) and perfused with a modified Krebs-Henseleit buffer (120.0 mM NaCl, 4.3 mM KCI, 1.1 mM KH₂PO₄, 2.4 mM CaCl₂, 1.3 mM MgCl₂, 13.14 mM glucose, 0.25 mM hydroxyethyl starch 200000/0.5, 25.0 mM NaHCO₃ adjusted to a constant pH range of 7.37-7.40, 800 mM L-arginine; Serag-Wissner GmbH & Co. KG, Naila, Germany) at a temperature of 37°C. Lung weight, right and left ventricle pressure, as well as ventilatory pressure were monitored and recorded during the whole experimental procedure. After 5-10 minutes, when the lung was properly flushed and all the parameters were stable, 5-amino-2,3-dihydro-1,4-phthalazinedione sodium salt was applied by adding 150 µl of a stock solution to 15 ml of the circulating perfusion buffer. The substance was applied 10 minutes prior to the first cigarette smoke application. Cigarette smoke was applied via trachea while lung is perfused with the buffer containing 5-amino-2,3-dihydro-1,4-phthalazinedione sodium salt. Cigarette smoke was prepared freshly before each application, by burning one cigarette (research cigarettes 3R4F, University of Kentucky, USA) in one minute using normoxic gas at the flow of 1l/min and collected in a 1 l glass bottle containing 5 g silica gel for removing the moist from the cigarette smoke. 50 ml of the cigarette smoke were taken via a syringe and applied to the lung via trachea (Figure 4A) in deep breaths (periodic inflation for 3-4 s) over a period of 5 min. The application was done manually while carefully monitoring the inspiratory pressure to avoid damage of the lung. The cigarette smoke application was repeated three times with a 1 hour break in-between.

Five treatment groups (n = 5, respectively) were investigated:

| | |
|---|---|
| A: | room air exposure |
| B: | cigarette smoke + diluent (buffer solution) |
| C: | cigarette smoke + 0.5 mM 5-amino-2,3-dihydro-1,4-phthalazinedione sodium salt |
| D: | cigarette smoke + 1 mM 5-amino-2,3-dihydro-1,4-phthalazinedione sodium salt |
| E: | cigarette smoke + 2 mM 5-amino-2,3-dihydro-1,4-phthalazinedione sodium salt |

5-amino-2,3-dihydro-1,4-phthalazinedione sodium salt Form I was dissolved in water for injection (vehicle) at the required concentrations described above. Stock solutions were prepared in water for injection. Further 1:100 dilutions were made with modified Krebs-Henseleit buffer (see above). Stock solutions were stored at -70°C in appropriate aliquots. The required amount of stock solution was thawed, and the corresponding working solution was prepared for immediate use.

One hour after the third cigarette smoke application lungs were removed from the system and fixed by inflating with formalin solution (via trachea) at the pressure of 12-15 cm H₂O for two hours at room temperature. Afterwards the fixed lungs were kept in PBS (phosphate buffer saline, see below) at +4°C until further dehydration and paraffin embedding. Paraffin blocks were cut 3 µm thick, dried overnight at 37°C and stained for 3-nitrotyrosine (3-NT).

The toxins and xenobiotics in the cigarette smoke lead to a dramatic increase in reactive oxygen species (ROS) and reactive nitrogen species (RNS). Oxidative and nitrosative stress correlate with the severity of inflammatory pulmonary diseases. They elevate the inflammatory response, cause a disbalance of proteolytic and anti-proteolytic activities, augment the number of apoptotic cells and decrease proliferation. These oxidants are able to overwhelm the antioxidant defenses and initiate inflammation by various mechanisms (Foronjy and D'Armiento (2006) Clinical and Applied Immunology Reviews 6: 53-72). The most potent RNS peroxynitrite (ONOO⁻) is formed by reaction between nitric oxide (NO) and superoxide anion radical (O₂⁻) (Szabo et al. (2007) Nat Rev Drug Discov 6: 662-680). ONOO⁻preferably attacks tyrosine residues in proteins to form the stable adduct 3-nitrotyrosine (Ricciardolo et al. (2004) Physiol Rev 84: 731-765; Seimetz et al. (2011) Cell 147: 293-305; Tsoumakidou et al. (2005) Chest 127: 1911-1918). Levels of 3-NT in sputum proteins have been found to negatively correlate with FEV1 in COPD patients (Ricciardolo et al. (2004) Physiol Rev 84: 731-765; Tsoumakidou et al. (2005) Chest 127: 1911-1918). Nitrated tyrosine residues alter cellular signalling, suggesting that 3-NT is not only a marker of nitrosative stress but may also have a functional relationship with the pathophysiology of inflammatory airway diseases (Davis et al. (2002); J Virol 76: 8347-8359; Murata and Kawanishi (2004) Biochem Biophys Res Comm 316: 123-128; Sugiura et al. (2004) Free Radic Res 38: 49-57;). It has been proposed that 3-NT contributes to airway hyper-responsiveness and epithelial damage (Tsoumakidou et al. (2005) Chest 127: 1911-1918) and plays a major role in the development of airway remodeling (Ichinose et al. (2000) Am J Respir Crit Care Med 162: 701-706).

The immunohistochemical staining of 3-nitrotyrosine was carried out according to the following protocol:

| **incubation time** | **reagent/condition** | **process** |
|---|---|---|
| 60 min | 59°C | deparaffinisation |
| 3x 10 min | xylol | |
| 2x 5 min | ethanol 99.6% | rehydratation |
| 5 min | ethanol 96% | |
| 5 min | ethanol 70% | |
| 20 min | 6% hydrogen peroxide in methanol | |
| 4 x 3 min | aqua dest | |
| 45 min | cooking in Rodent decloaker buffer (10x) | antigen retrieval |
| 30 min | cooling down | |
| wash | aqua dest | |
| 2x 5 min | PBS pH 7.4 | |
| 60 min | 10% BSA + 1:1000 DR Fc block | |
| 4x 5 min | PBS pH 7.4 | blocking |
| 30 min | rodent M | |
| 2x 5 min | PBS pH 7.4 | |
| overnight +4°C | primary antibody (nTyr Sigma) 1:200 DR | |
| 4x 5 min | TBS pH 7.2 | |
| 20 min | post block AP | |
| 2x 5 min | TBS pH 7.2 | |
| 30 min | polymer AP kit (rabbit/mouse) | |
| 3x 5 min | TBS pH 7.2 | |
| 1 min | aqua dest | staining |
| 5-10 min | Warp Red | |
| wash | TBS pH 7.2 | |
| 2 min | hematoxylin 1:10 DR | |
| wash | aqua dest | |
| 1 min | PBS pH 7.4 | |
| 5 min | DAPI in PBS 1:1000 | |
| 2x 2min | PBS pH 7.4 | |
| - | Dako Fluorescent Mounting Medium | covering |

Xylol was purchased from Carl Roth GmbH + Co.KG, Karlsruhe, Germany. Ethanol (96 % and 99.6 %) was purchased from Otto Fischar GmbH % Co.KG, Saarbrücken, Germany. Ethanol (70 %) was purchased from SAV Liquid Production GmbH, Flintsbach am Inn, Germany. Hydrogen peroxide was purchased from Merck KGaA, Darmstadt, Germany. Methanol, Bovine Serum Albumin (BSA), DAPI (4',6-diamidino-2-phenylidone) and Anti-Nitrotyrosine Antibody (N0409; batch: 120M4825) were purchased from Sigma-Aldrich Co., Darmstadt, Germany. Rodent decloaker buffer (10x) and Warp Red Chromogen Kit were purchased from Biocare Medical, Pacheco, Ca., USA. Tris wash buffer (TBS), CAT hematoxylin staining solution and AP Polymer System (mouse/rabbit) were purchased from Zytomed Systems GmbH, Berlin, Germany. Dako Fluorescent Mounting Medium was purchased from Dako North America Inc., Via Real Carpinteria, Ca., USA. TruStain fcX (antimouse CD16/32; DR Fc block) was purchased from BioLegend Inc., San Diego, Ca., USA. PBS (phosphate-buffered saline) was prepared with 8 g/l sodium chloride (Carl Roth GmbH + Co.KG, Karlsruhe, Germany), 0.2 g/l potassium chloride (Carl Roth GmbH + Co.KG, Karlsruhe, Germany), 1.42 g/l disodium hydrogenphosphate (Merck KGaA, Darmstadt, Germany) and 0.27 g/l potassium dihydrogenphosphate (Merck KGaA, Darmstadt, Germany).

Stained histological samples were blindly analysed by light microscope. 3-nitrotyrosine levels in the lung parenchyma were quantified as a percentage of stained surface area. Quantification was performed under 200x magnification in 5-10 randomly selected fields with exclusion of large bronchi and vessels. One-Way ANOVA statistical test with Bonferroni correction was performed for comparison between groups. Differences with p < 0.05 were considered statistically significant.

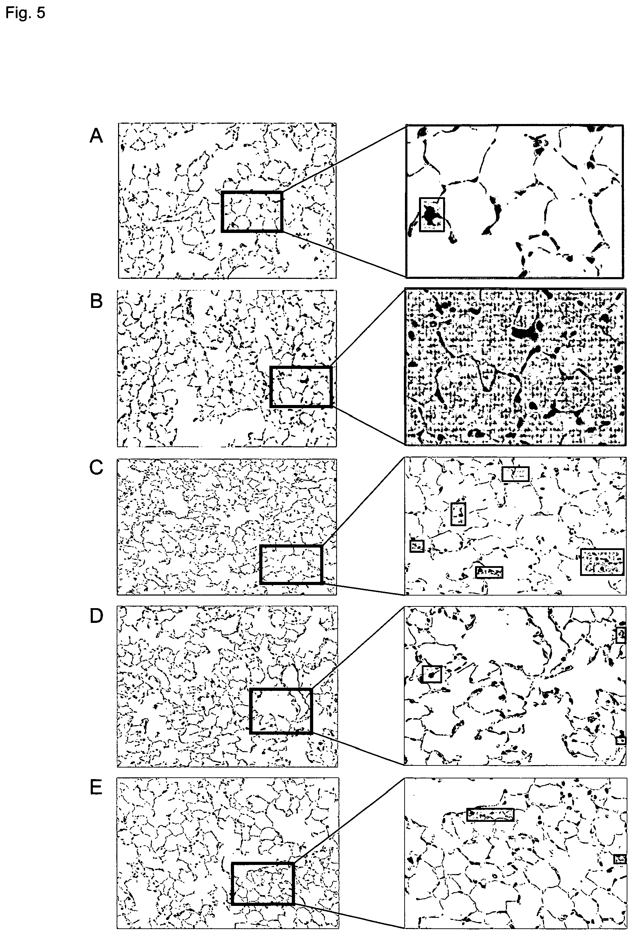

Cigarette smoke applied via trachea lead to a significant increase in 3-nitrotyrosine in septa of the exposed lungs (Fig. 5B), in comparison to room air as control (Fig. 5A). 5-amino-2,3-dihydro-1,4-phthalazinedione sodium salt was added to the perfusing buffer prior to the cigarette smoke application and was kept during the whole experiment. Cigarette smoke induced 3-nitrotyrosine formation could be almost completely abolished in the lungs perfused with buffer containing 1 mM 5-amino-2,3-dihydro-1,4-phthalazinedione sodium salt (Fig. 5D) or 2 mM 5-amino-2,3-dihydro-1,4-phthalazinedione sodium salt (Fig. 5E), whereas the lowest 5-amino-2,3-dihydro-1,4-phthalazinedione sodium salt concentration (0.5 mM; Fig. 5C) yielded a moderate effect.

Quantification of the staining:

| **group** | **mean ± SEM [% area]** |
|---|---|
| A: room air | 0.24 ± 0.09 |
| B: cigarette smoke | 3.07 ± 0.39 |
| C: cigarette smoke + 0.5 mM | 2.24 ± 0.28 |
| 5-amino-2,3-dihydro-1,4-phthalazinedione sodium salt | |
| D: cigarette smoke + 1 mM | 0.72 ± 0.26 |
| 5-amino-2,3-dihydro-1,4-phthalazinedione sodium salt | |
| E: cigarette smoke + 2 mM | 0.37 ± 0.13 |
| 5-amino-2,3-dihydro-1,4-phthalazinedione sodium salt | |

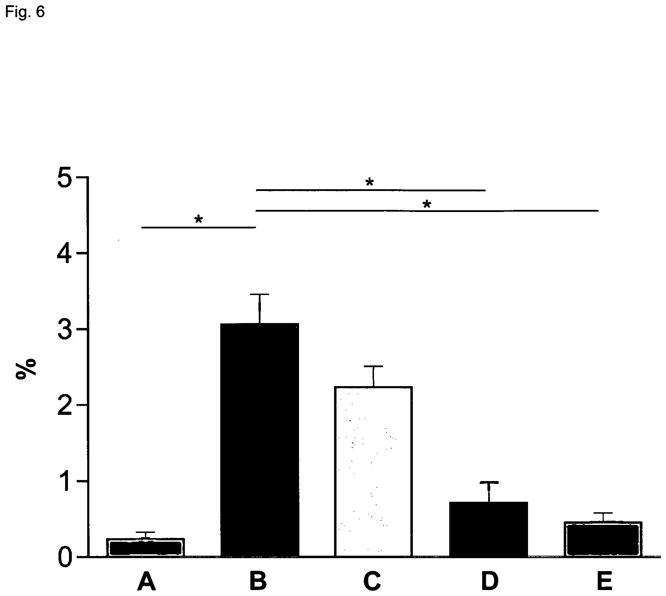

The results are depicted as bar diagrams in Fig. 6. The values (mean ± SEM) indicate the percentage of the stained surface in the evaluated histological samples (5 mice per group; 5 - 6 evaluated histological samples per mouse).

From this experiment it can be concluded that pre-treatment with 5-amino-2,3-dihydro-1,4-phthalazinedione sodium salt prevents cigarette smoke-induced 3-nitrotyrosine formation in the lung parenchyma in the ILU model.

This suggests that 5-amino-2,3-dihydro-1,4-phthalazinedione sodium salt has a protective effect against acute cigarette smoke-induced lung injury. Thus, these results can be regarded as predictive for a beneficial effect of 5-amino-2,3-dihydro-1,4-phthalazinedione and its pharmaceutically acceptable salts in the inhalatory prophylaxis and/or treatment of all inflammatory pulmonary diseases.

### Example 4: 5-amino-2,3-dihydro-1,4-phthalazinedione sodium salt inhibits the replication of SARS-CoV-2 in infected Vero B4 cells

In order to investigate whether 5-amino-2,3-dihydro-1,4-phthalazinedione sodium salt has an effect on the spread of viral infection, Western Blot (WB) analyses were carried out. Vero B4 cells (National Institute of Health, Bethesda, USA; Meyer et al. (2015) Emerg Infect Dis 21: 181-182) were maintained in Dulbecco's Modified Eagle's Medium (DMEM) containing 10% (v/v) inactivated fetal calf serum (FCS), 2 mM L-glutamine, 100 U/mL penicillin, and 100 µg/mL streptomycin. Confluent monolayers of Vero B4 cells were infected for one hour in FCS-free DMEM with a 100-fold dilution of the field isolate SARS-CoV-2_{PR-1} (isolated from a 61 years old patient six days after the presumed date of infection and two days after start of mild COVID-19 symptoms). Cells were then washed with PBS (phosphate buffer saline), provided with fresh medium containing 5-amino-2,3-dihydro-1,4-phthalazinedione sodium salt in non-cytotoxic concentrations (0.5 µM, 1 µM, 2 µM). The treatment with 5-amino-2,3-dihydro-1,4-phthalazinedione sodium salt was carried out over the entire experimental procedure. 3 days post infection (dpi) virus-containing cell culture supernatants were harvested. Virions were purified from cell culture supernatants via a 20% (w/v) sucrose cushion (20,000 × g, 4°C, 90 min). Cells were washed with PBS and the pellet was dissolved

in SDS (sodium dodecyl sulfate) sample buffer, separated by SDS-PAGE gel electrophoresis, transferred onto nitrocellulose membranes and blocked with 3% bovine serum albumin, and incubated with the appropriate primary antibody. SARS-CoV-2 protein was visualized using a convalescent SARS-CoV-2 patient serum. The anti-human secondary antibody coupled to horseradish peroxidase was obtained from Dianova (Hamburg, Germany). Visualization was effected by means of an electrochemiluminescence reaction.

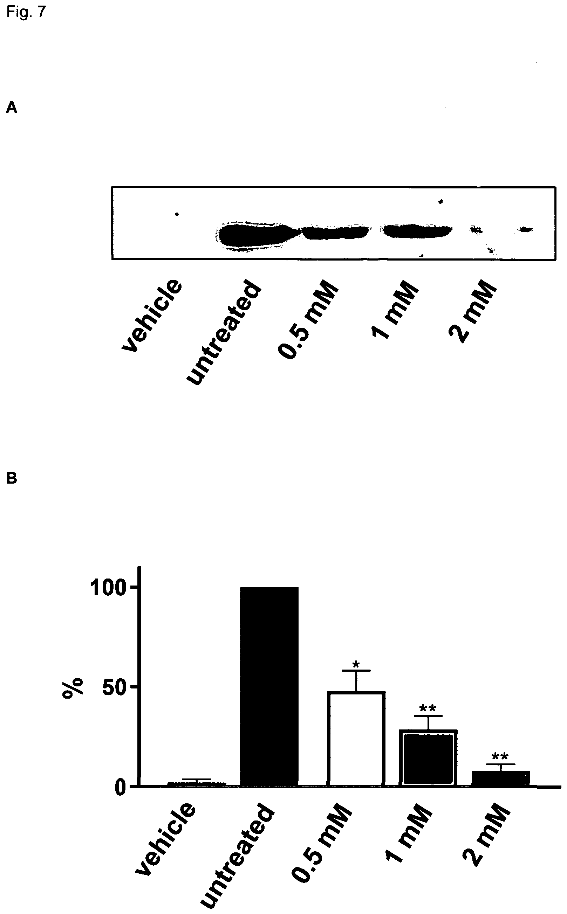

Herein, an inhibition of SARS-Cov-2 replication was shown in Vero B4 cells. 5-amino-2,3-dihydro-1,4-phthalazinedione sodium salt showed a clear reduction of SARS-CoV-2 nucleocapsid protein. Respective gel bands are shown in Fig. 7A.

Densitometric evaluations of SARS-CoV-2 nucleocapsid were carried out with the analysis program AIDA^{®}. Densitometric evaluation allows for the quantification of signal intensities in Western Blot and thus for conclusions on the quantity of a certain protein in the sample. The evaluation clearly showed that after the addition of 5-amino-2,3-dihydro-1,4-phthalazinedione sodium salt the generation of SARS-CoV-2 proteins was inhibited dose-dependently (Fig. 7B). Statistical analyses were performed using unpaired T Test with Welch's correction; **p<0.01, *p<0.05.

### Example 5: In effective concentrations 5-amino-2,3-dihydro-1,4-phthalazinedione sodium salt is not cytotoxic in Vero B4 cell cultures

For addressing the question whether 5-amino-2,3-dihydro-1,4-phthalazinedione sodium salt shows a cytotoxic effect in the abovementioned systems non-infected Vero B4 cells were treated in parallel to the Western blot studies with increasing concentrations of 5-amino-2,3-dihydro-1,4-phthalazinedione sodium salt (0.25 mM, 0.5 mM, 1 mM, 2 mM, 4 mM). Toxicity is assessed with a WST (water-soluble tetrazolium salt 1) assay. Herein viable cells with an intact mitochondrial succinate-tetrazolium dehydrogenase system effect an enzymatic conversion of the feebly red tetrazolium salt WST-1 (4-[3-(4-iodophenyl)-2-(4-nitrophenyl)-2H-5-tetrazolio]-1,3 benzene disulfonate) into dark red formazan. This color change can be measured photometrically in a spectrophotometer. Thus, the WST assay is a very sensitive method for measuring the toxicity of substances on the cell metabolism. The value for untreated cells was set to 100%.

It can be shown that 5-amino-2,3-dihydro-1,4-phthalazinedione sodium salt does not display any significant toxic effect in antivirally effective concentrations in Vero B4 cells during an observation period of 3 days.

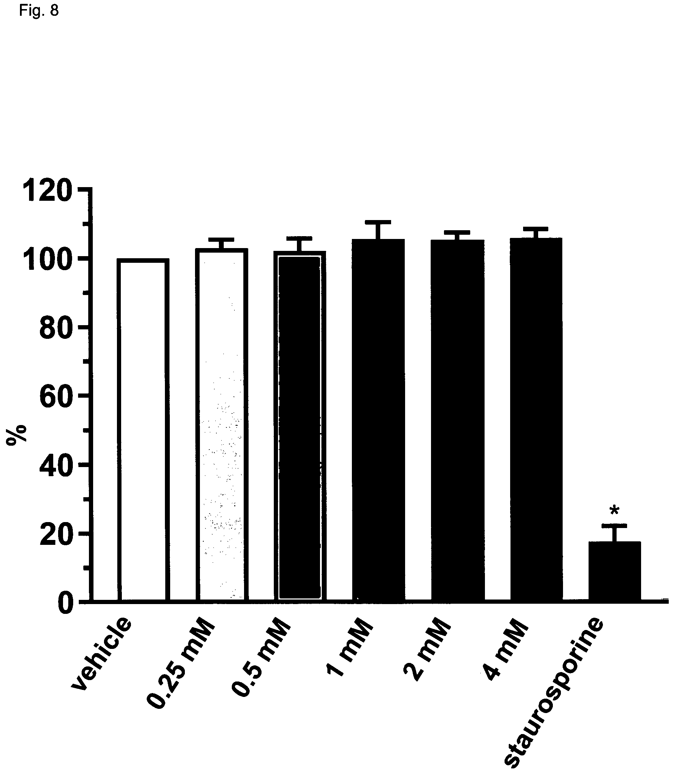

In Fig. 8 the percentage of viable cells is depicted in comparison to untreated cells. The value for untreated cells was set to 100%. 1 µM staurosporine (an indolocarbazole compound from *Streptomyces staurosporeus,* an apoptosis inducer) was used as positive control. Statistical analyses were performed using unpaired T Test with Welch's correction; **p<0.01, *p<0.05.

Thus, it can be stated that the antiviral effect of 5-amino-2,3-dihydro-1,4-phthalazinedione sodium salt is not due to unspecific cytotoxic effects.

### FIGURES

Fig. 1: Percentual reduction of the secretion of four pro-inflammatory cytokines after treatment with 5-amino-2,3-dihydro-1,4-phthalazinedione sodium salt. LPS induction only was set to 100 %.
Blocks from left to right: TNF-alpha, IL-6, IL-12, IL-1-beta
Bars from left to right, respectively:
   white: untreated control
   black: treatment with LPS only
   dark grey: LPS + 1 mM 5-amino-2,3-dihydro-1,4-phthalazinedione sodium salt
   light grey: LPS + 0.5 mM 5-amino-2,3-dihydro-1,4-phthalazinedione sodium salt
   *: significant; p < 0.05

Fig. 2: Percentual survival rate of mice in days after sepsis-induction in the PCI model
A: therapeutic treatment (daily intraperitoneal injection of 5-amino-2,3-dihydro-1,4-phthalazinedione sodium salt)
B: prophylactic treatment (intraperitoneal injection of 5-amino-2,3-dihydro-1,4-phthalazinedione sodium salt 3 hours before and 6 hours after sepsis induction)

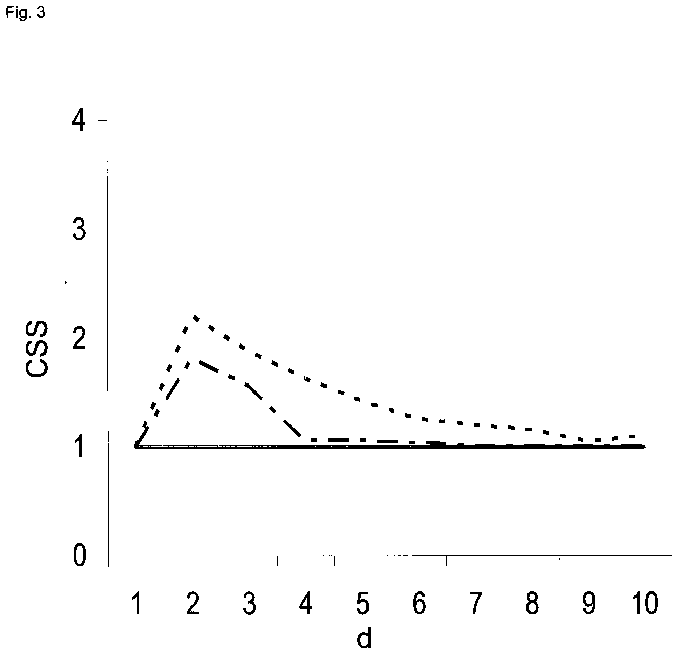

― control
- - - - - PCI
―- PCI + 2 mg/kg 5-amino-2,3-dihydro-1,4-phthalazinedione sodium salt
―― PCI + 5 mg/kg 5-amino-2,3-dihydro-1,4-phthalazinedione sodium salt
% percentual survival rate
d days after sepsis induction
Fig. 3: Clinical severity score of mice in days after sepsis-induction in the PCI model
― control
- - - - - PCI
―- PCI + 5 mg/kg 5-amino-2,3-dihydro-1,4-phthalazinedione sodium salt
CSS clinical severity score
d days after sepsis induction
Fig. 4:
A: Schematic drawing of the experimental setup of Example 3
   1 - cigarette smoke
   2 - ventilator
   3 - trachea
   4 - lung
   5 - heart
   6 - reservoir
   7 - aqueous solution of 5-amino-2,3-dihydro-1,4-phthalazinedione sodium salt
   8 - roller pump
B: Picture of the experimental setup of Example 3

Fig. 5: Immunohistochemical staining of representative samples from Example 3

| | |
|---|---|
| Left panel: | 200 x magnification |
| Right panel: | 400 x magnification, enlarged detail from the left panel |

A: room air
B: cigarette smoke + diluent (buffer solution)
C: cigarette smoke + 0.5 mM 5-amino-2,3-dihydro-1,4-phthalazinedione sodium salt
D: cigarette smoke + 1 mM 5-amino-2,3-dihydro-1,4-phthalazinedione sodium salt
E: cigarette smoke + 2 mM 5-amino-2,3-dihydro-1,4-phthalazinedione sodium salt
In the right panel the inflamed areas are highlighted.
Fig. 6: Statistical evaluation of the immunohistochemical staining of samples from Example 3. The percentage of stained surface area corresponds to the grade of inflammation (n = 5; mean ± SEM). Bars marked with an asterisk indicate a highly significant difference between two groups (p < 0.001).
A: room air
B: cigarette smoke + diluent (buffer solution)
C: cigarette smoke + 0.5 mM 5-amino-2,3-dihydro-1,4-phthalazinedione sodium salt
D: cigarette smoke + 1 mM 5-amino-2,3-dihydro-1,4-phthalazinedione sodium salt
E: cigarette smoke + 2 mM 5-amino-2,3-dihydro-1,4-phthalazinedione sodium salt

Fig. 7:
A: Western Blot bands of SARS-CoV-2 nucleocapsid after 3d treatment with 0.5 mM, 1 mM and 2 mM 5-amino-2,3-dihydro-1,4-phthalazinedione sodium salt, respectively, vs. vehicle and untreated cells.
B: Densitometric evaluation of SARS-CoV-2 nucleocapsid detected in Western Blot bands after 3d treatment with 0.5 mM, 1 mM and 2 mM 5-amino-2,3-dihydro-1,4-phthalazinedione sodium salt, respectively, vs. vehicle and untreated cells. The percentage of the detected viral protein is indicated. Vehicle was set to 100%. (mean ± SEM; n = 3/group; each in duplicates; **p<0.01, *p<0.05)

Fig. 8: Cell viability in the WST assay after 3d treatment with different concentrations of 5-amino-2,3-dihydro-1,4-phthalazinedione sodium salt. The percentage of viable cells is indicated. Untreated cells were taken as 100%. Staurosporine (1 µM) was used as positive control. (mean ± SEM; n = 3/group; each in duplicates)

## Claims

1. 5-amino-2,3-dihydro-1,4-phthalazinedione or one of its pharmaceutically acceptable salts for use in the prophylaxis or treatment of acute lung injury, wherein the acute lung injury has been caused by a coronaviral infection by SARS-CoV, SARS-CoV-2 or MERS.

2. 5-amino-2,3-dihydro-1,4-phthalazinedione or one of its pharmaceutically acceptable salts for use according to claim 1, wherein the pharmaceutically acceptable salt of 5-amino-2,3-dihydro-1,4-phthalazinedione is 5-amino-2,3-dihydro-1,4-phthalazinedione sodium salt.

3. 5-amino-2,3-dihydro-1,4-phthalazinedione or one of its pharmaceutically acceptable salts for use according to claim 2, wherein 5-amino-2,3-dihydro-1,4-phthalazinedione sodium salt is provided as one of crystalline anhydrate polymorph forms I, II or III **characterized by** crystallography values determined by means of x-ray powder diagrams:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3 for form I,
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8 for form II, and
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93 for form III.

4. A pharmaceutical composition for use in the prophylaxis or treatment of acute lung injury caused by a coronaviral infection by SARS-CoV, SARS-CoV-2 or MERS, wherein said composition contains 5-amino-2,3-dihydro-1,4-phthalazinedione or one of its pharmaceutically acceptable salts, a carrier and at least one pharmaceutically acceptable excipient.

5. A composition for use according to claim 4, wherein the at least one pharmaceutically acceptable excipient is selected from a group comprising binding agents, colorants, buffers, preservatives, antioxidants, coatings, sweeteners, thickening agents, pH-regulators, acidity regulators acidifiers, solvents, isotonizing agents, disintegrants, glidants, lubricants, emulsifiers, solubilizing agents, stabilizers, diluents, anti-caking agents, sorbents, foaming agents, anti-foaming agents, opacifiers, fatliquors, consistency enhancers, hydrotropes, aromatic and flavoring substances.

6. Combination of 5-amino-2,3-dihydro-1,4-phthalazinedione or one of its pharmaceutically acceptable salts and at least one active agent selected from a group comprising steroidal and non-steroidal anti-inflammatory drugs, immunomodulators, immunosuppressive agents; anti-infective agents like antibiotics, antiretroviral agents, antiviral agents, antifungal agents and antiprotozoal agents, analgesics, anticoagulants, antiplatelet drugs, bronchodilators, pulmonary vasodilators, mucolytic agents, pulmonary surfactants, antioxidants, ENaC-activating agents, HMG-CoA reductase inhibitors, calcium antagonists or AT₁ receptor antagonists for use in the prophylaxis or treatment of acute lung injury caused by a coronaviral infection by SARS-CoV, SARS-CoV-2 or MERS.

7. 5-amino-2,3-dihydro-1,4-phthalazinedione or one of its pharmaceutically acceptable salts, a composition as defined in any one of claims 4 or 5 or a combination as defined in claim 6 for use in the treatment of acute lung injury caused by a coronaviral infection by SARS-CoV, SARS-CoV-2 or MERS, wherein a previous treatment with at least one other pharmaceutically active agent was refractory.

8. 5-amino-2,3-dihydro-1,4-phthalazinedione or one of its pharmaceutically acceptable salts, a composition as defined in any one of claims 4 or 5 or a combination as defined in claim 6 for use in the prophylaxis or treatment of acute lung injury caused by a coronaviral infection by SARS-CoV, SARS-CoV-2 or MERS, wherein said substance, composition or combination is applied orally in the form of tablets, soft gelatin capsules, hard gelatin capsules, sugar-coated tablets, pills, powders, granulates, juices, syrups, drops, teas, solutions or suspensions in aqueous or non-aqueous liquids, edible foams, mousses, oil-in-water emulsions or water-in-oil emulsions.

9. 5-amino-2,3-dihydro-1,4-phthalazinedione or one of its pharmaceutically acceptable salts, a composition as defined in any one of claims 4 or 5 or a combination as defined in claim 6 for use in the prophylaxis or treatment of acute lung injury caused by a coronaviral infection by SARS-CoV, SARS-CoV-2 or MERS, wherein said substance, composition or combination is applied parenterally in the form of intravenous injection, intraarterial injection or intraperitoneal injection.

10. 5-amino-2,3-dihydro-1,4-phthalazinedione or one of its pharmaceutically acceptable salts, a composition as defined in any one of claims 4 or 5 or a combination as defined in claim 6 for use in the prophylaxis or treatment of acute lung injury caused by a coronaviral infection by SARS-CoV, SARS-CoV-2 or MERS, wherein said substance, composition or combination is applied by inhalation by using a vibrant mesh nebulizer, metered dose-inhaler or dry-powder inhaler.

11. 5-amino-2,3-dihydro-1,4-phthalazinedione or one of its pharmaceutically acceptable salts, a composition as defined in any one of claims 4 or 5 or a combination as defined in claim 6 for use in the prophylaxis or treatment of acute lung injury caused by a coronaviral infection by SARS-CoV, SARS-CoV-2 or MERS, wherein said substance, composition or combination is added to the ventilation air of a cardiopulmonary bypass device.

12. 5-amino-2,3-dihydro-1,4-phthalazinedione or one of its pharmaceutically acceptable salts, a composition as defined in any one of claims 4 or 5 or a combination as defined in claim 6 for use in the prophylaxis or treatment of acute lung injury caused by a coronaviral infection by SARS-CoV, SARS-CoV-2 or MERS, wherein said substance, composition or combination is applied in form of liposomes, micelles, multilamellar vesicles or a cyclodextrin complex.

## Patentansprüche

1. 5-Amino-2,3-dihydro-1,4-phthalazindion oder eines seiner pharmazeutisch verträglichen Salze zur Verwendung in der Prophylaxe oder der Behandlung von akutem Lungenversagen, wobei das akute Lungenversagen durch eine coronavirale Infektion mit SARS-CoV, SARS-CoV-2 oder MERS verursacht wurde.

2. 5-Amino-2,3-dihydro-1,4-phthalazindion oder eines seiner pharmazeutisch verträglichen Salze zur Verwendung gemäß Anspruch 1, wobei das pharmazeutisch verträgliche Salz von 5-Amino-2,3-dihydro-1,4-phthalazindion 5-Amino-2,3-dihydro-1,4-phthalazindion Natriumsalz ist.

3. 5-Amino-2,3-dihydro-1,4-phthalazindion oder eines seiner pharmazeutisch verträglichen Salze zur Verwendung gemäß Anspruch 2, wobei 5-Amino-2,3-dihydro-1,4-phthalazindion Natriumsalz als einer der kristallinen Anhydratpolymorphe Form I, II oder III zur Verfügung gestellt wird, die durch Röntgenpulverdiffraktometrie-Diagramme bestimmten kristallographischen Werte charakterisiert sind:
d-Werte: 13,5; 6,9; 5,2; 4,6; 3,9; 3,5; 3,4; 3,3; 3,1; 3,0 und/oder
2-Theta-Werte: 6,5; 12,7; 16,9; 19,3; 22,8; 25,8; 26,6; 27,2; 28,7; 30,3 für Form I,
d-Werte: 12,9; 7,9; 7,1; 6,5; 5,3; 4,0; 3,7; 3,6; 3,3; 3,2 und/oder
2-Theta-Werte: 6,8; 11,2; 12,5; 13,7; 16,7; 22,4; 24,3; 24,9; 27,2; 27,8 für Form II, und
d-Werte: 13,131; 7,987; 7,186; 6,566; 6,512; 5,372; 3,994; 3,662; 3,406; 3,288; 3,283; 3,222; 3,215; 3,127; 2,889 und/oder
2-Theta-Werte: 6,73; 11,07; 12,31; 13,48; 13,59; 16,49; 22,24; 24,29; 26,14; 27,10; 27,14; 27,67; 27,72; 28,52; 30,93 für Form III.

4. Eine pharmazeutische Zusammensetzung zur Verwendung in der Prophylaxe oder der Behandlung von akutem Lungenversagen, das durch eine coronavirale Infektion mit SARS-CoV, SARS-CoV-2 oder MERS verursacht wurde, wobei die besagte Zusammensetzung 5-Amino-2,3-dihydro-1,4-phthalazindion oder eines seiner pharmazeutisch verträglichen Salze, einen Trägerstoff und mindestens einen pharmazeutisch verträglichen Hilfsstoff enthält.

5. Eine Zusammensetzung zur Verwendung gemäß Anspruch 4, wobei der mindestens eine pharmazeutisch verträgliche Hilfsstoff aus einer Gruppe ausgewählt wird, die Bindemittel, Farbstoffe, Puffer, Konservierungsmittel, Antioxidantien, Beschichtungen, Süßungsmittel, Verdickungsmittel, pH-Regulatoren, Säureregulatoren, Säuerungsmittel, Lösungsmittel, isotonische Agentien, Sprengmittel, Gleitmittel, Schmiermittel, Emulgatoren, Lösungsvermittler, Stabilisierungsmittel, Verdünnungsmittel, Antibackmittel, Sorbenzien, Schaumbildner, Antischaummittel, Trübungsmittel, Fettungsmittel, Konsistenzverstärker, Hydrotrope, Aromastoffe und Geschmacksstoffe umfasst.

6. Kombination aus 5-Amino-2,3-dihydro-1,4-phthalazindion oder einem seiner pharmazeutisch verträglichen Salze und mindestens einem Wirkstoff, ausgewählt aus einer Gruppe, die steroidale und nichtsteroidale Entzündungshemmer, Immunmodulatoren, Immunsuppressiva, Antiinfektiva wie Antibiotika, antiretrovirale Wirkstoffe, antivirale Wirkstoffe, Antimykotika und Antiprotozoenmittel, Analgetika, Antikoagulanzien, Thrombozytenaggregationshemmer, Bronchodilatatoren, pulmonale Vasodilatatoren, Mukolytika, pulmonale Surfactant-Ergänzungsmittel, Antioxidantien, ENaC-aktivierende Mittel, HMG-CoA-Reduktase-Inhibitoren, Calciumantagonisten oder AT₁-Rezeptorantagonisten zur Verwendung bei der Prophylaxe oder Behandlung von akutem Lungenversagen umfasst, das durch eine coronavirale Infektion mit SARS-CoV, SARS-CoV-2 oder MERS verursacht wurde.

7. 5-Amino-2,3-dihydro-1,4-phthalazindion oder eines seiner pharmazeutisch verträglichen Salze, eine Zusammensetzung gemäß einem der Ansprüche 4 oder 5 oder eine Kombination gemäß Anspruch 6 zur Verwendung bei der Behandlung von akutem Lungenversagen, das durch eine Coronavirus-Infektion mit SARS-CoV, SARS-CoV-2 oder MERS verursacht wurde, wobei eine vorherige Behandlung mit mindestens einem anderen pharmazeutisch wirksamen Mittel refraktär war.

8. 5-Amino-2,3-dihydro-1,4-phthalazindion oder eines seiner pharmazeutisch verträglichen Salze, eine Zusammensetzung gemäß einem der Ansprüche 4 oder 5 oder eine Kombination gemäß Anspruch 6 zur Verwendung bei der Prophylaxe oder Behandlung von akutem Lungenversagen, das durch eine Coronavirus-Infektion mit SARS-CoV, SARS-CoV-2 oder MERS verursacht wurde, wobei die besagte Substanz, Zusammensetzung oder Kombination oral in Form von Tabletten, Weichgelatinekapseln, Hartgelatinekapseln, Dragées, Pillen, Pulvern, Granulaten, Säften, Sirupen, Tropfen, Tees, Lösungen oder Suspensionen in wässrigen oder nichtwässrigen Flüssigkeiten, essbaren Schäumen, Mousses, Öl-in-Wasser-Emulsionen oder Wasser-in-Öl-Emulsionen verabreicht wird.

9. 5-Amino-2,3-dihydro-1,4-phthalazindion oder eines seiner pharmazeutisch verträglichen Salze, eine Zusammensetzung gemäß einem der Ansprüche 4 oder 5 oder eine Kombination gemäß Anspruch 6 zur Verwendung bei der Prophylaxe oder Behandlung von akutem Lungenversagen, das durch eine Coronavirus-Infektion mit SARS-CoV, SARS-CoV-2 oder MERS verursacht wurde, wobei die besagte Substanz, Zusammensetzung oder Kombination parenteral in Form einer intravenösen Injektion, einer intraarteriellen Injektion oder einer intraperitonealen Injektion verabreicht wird.

10. 5-Amino-2,3-dihydro-1,4-phthalazindion oder eines seiner pharmazeutisch verträglichen Salze, eine Zusammensetzung gemäß einem der Ansprüche 4 oder 5 oder eine Kombination gemäß Anspruch 6 zur Verwendung bei der Prophylaxe oder Behandlung von akutem Lungenversagen, das durch eine Coronavirus-Infektion mit SARS-CoV, SARS-CoV-2 oder MERS verursacht wurde, wobei die besagte Substanz, Zusammensetzung oder Kombination durch Inhalation unter Verwendung eines Schwingmembran-Verneblers, eines Fixed-Dose-Inhalators oder eines Trockenpulverinhalators verabreicht wird.

11. 5-Amino-2,3-dihydro-1,4-phthalazindion oder eines seiner pharmazeutisch verträglichen Salze, eine Zusammensetzung gemäß einem der Ansprüche 4 oder 5 oder eine Kombination gemäß Anspruch 6 zur Verwendung bei der Prophylaxe oder Behandlung von akutem Lungenversagen, das durch eine Coronavirus-Infektion mit SARS-CoV, SARS-CoV-2 oder MERS verursacht wurde, wobei die besagte Substanz, Zusammensetzung oder Kombination der Beatmungsluft einer Herz-Lungen-Bypass-Vorrichtung zugesetzt wird.

12. 5-Amino-2,3-dihydro-1,4-phthalazindion oder eines seiner pharmazeutisch verträglichen Salze, eine Zusammensetzung gemäß einem der Ansprüche 4 oder 5 oder eine Kombination gemäß Anspruch 6 zur Verwendung bei der Prophylaxe oder Behandlung von akutem Lungenversagen, das durch eine Coronavirus-Infektion mit SARS-CoV, SARS-CoV-2 oder MERS verursacht wurde, wobei die besagte Substanz, Zusammensetzung oder Kombination in Form von Liposomen, Mizellen, multilamellaren Vesikeln oder eines Cyclodextrin-Komplexes verabreicht wird.

## Revendications

1. 5-amino-2,3-dihydro-1,4-phtalazinedione ou l'un de ses sels pharmaceutiquement acceptables pour l'utilisation dans la prophylaxie ou le traitement d'une insuffisance respiratoire aiguë, dans lequel l'insuffisance respiratoire aiguë a été causée par une infection à coronavirus par le SARS-CoV, le SARS-CoV-2 ou le MERS.

2. 5-amino-2,3-dihydro-1,4-phtalazinedione ou l'un de ses sels pharmaceutiquement acceptables pour l'utilisation selon la revendication 1, dans lequel le sel pharmaceutiquement acceptable de 5-amino-2,3-dihydro-1,4-phtalazinedione est le sel de sodium 5-amino-2,3-dihydro-1,4-phthalazinedione.

3. 5-amino-2,3-dihydro-1,4-phtalazinedione ou l'un de ses sels pharmaceutiquement acceptables pour l'utilisation selon la revendication 2, dans lequel le sel de sodium de 5-amino-2,3-dihydro-1,4-phthalazinedione est fourni sous l'une des Formes polymorphes anhydriques cristallines I, II ou III **caractérisées par** des valeurs cristallographiques déterminées au moyen de diagrammes de poudres aux rayons X :
valeurs d: 13,5; 6,9; 5,2; 4,6; 3,9; 3,5; 3,4; 3,3; 3,1; 3,0 et/ou
valeurs 2-thêta: 6,5; 12,7; 16,9; 19,3; 22,8; 25,8; 26,6; 27,2; 28,7; 30,3 pour la Forme I,
valeurs d: 12,9; 7,9; 7,1; 6,5; 5,3; 4,0; 3,7; 3,6; 3,3; 3,2 et/ou
valeurs 2-thêta: 6,8; 11,2; 12,5; 13,7; 16,7; 22,4; 24,3; 24,9; 27,2; 27,8 pour la Forme II, et
valeurs d: 13,131; 7,987; 7,186; 6,566; 6,512; 5,372; 3,994; 3,662; 3,406; 3,288; 3,283; 3,222; 3,215; 3,127; 2,889 et/ou
valeurs 2-thêta: 6,73; 11,07; 12,31; 13,48; 13,59; 16,49; 22,24; 24,29; 26,14; 27,10; 27,14; 27,67; 27,72; 28,52; 30,93 pour la Forme III.

4. Composition pharmaceutique pour l'utilisation dans la prophylaxie ou le traitement d'une insuffisance respiratoire aiguë, dans lequel l'insuffisance respiratoire aiguë a été causée par une infection à coronavirus par le SARS-CoV, le SARS-CoV-2 ou le MERS, où ladite composition contient de la 5-amino-2,3-dihydro-1,4-phtalazinedione ou l'un de ses sels pharmaceutiquement acceptables, un véhicule et au moins un excipient pharmaceutiquement acceptable.

5. Composition à utiliser selon la revendication 4, dans laquelle au moins un excipient pharmaceutiquement acceptable est choisi dans un groupe comprenant des liants, colorants, tampons, conservateurs, antioxydants, revêtements, édulcorants, épaississants, régulateurs de pH, régulateurs d'acidité, acidifiants, solvants, agents isotoniques, désintégrants, agents de glissement, lubrifiants, émulsifiants, agents de solubilisation, stabilisants, diluants, agents anti-agglomérants, sorbants, agents moussants, agents anti-moussants, opacifiants, fatliquors, améliorateurs de consistance, hydrotropes, substances aromatiques et aromatisants.

6. Combinaison de 5-amino-2,3-dihydro-1,4-phtalazinedione ou de l'un de ses sels pharmaceutiquement acceptables et d'au moins un agent actif choisi dans un groupe comprenant des anti-inflammatoires stéroïdiens et non stéroïdiens, immunomodulateurs, agents immunosuppresseurs, agents anti-infectieux tels que des antibiotiques, agents antirétroviraux, agents antiviraux, agents antifongiques et agents antiprotozoaires, analgésiques, anticoagulants, médicaments antiplaquettaires, bronchodilatateurs, vasodilatateurs pulmonaires, agents mucolytiques, surfactants pulmonaires, antioxydants, agents activateurs de l'ENaC, inhibiteurs de l'HMG-CoA réductase, antagonistes du calcium ou antagonistes du récepteur AT₁, destinés à être utilisés dans la prophylaxie ou le traitement d'une insuffisance respiratoire aiguë causée par une infection à coronavirus par le SARS-CoV, le SARS-CoV-2 ou le MERS.

7. 5-amino-2,3-dihydro-1,4-phthalazinedione ou l'un de ses sels pharmaceutiquement acceptables, une composition telle que définie dans l'une des revendications 4 ou 5 ou une combinaison telle que définie dans la revendication 6 pour le traitement d'une insuffisance respiratoire aiguë causée par une infection à coronavirus par le SARS-CoV, le SARS-CoV-2 ou le MERS, lorsqu'un traitement antérieur avec au moins un autre agent pharmaceutiquement actif s'est révélé réfractaire.

8. 5-amino-2,3-dihydro-1,4-phthalazinedione ou l'un de ses sels pharmaceutiquement acceptables, une composition telle que définie dans l'une des revendications 4 ou 5 ou une combinaison telle que définie dans la revendication 6 pour utilisation dans la prophylaxie ou le traitement d'une insuffisance respiratoire aiguë causée par une infection à coronavirus par le SARS-CoV, le SARS-CoV-2 ou le MERS, dans laquelle ladite substance, composition ou combinaison est appliquée par voie orale sous forme de comprimés, de capsules de gélatine molle, de capsules de gélatine dure, de comprimés enrobés de sucre, de pilules, de poudres, de granulés, de jus, de sirops, de gouttes, d'infusions, de solutions ou de suspensions dans des liquides aqueux ou non aqueux, de mousses comestibles, de mousses, d'émulsions d'huile dans l'eau ou d'émulsions d'eau dans l'huile.

9. 5-amino-2,3-dihydro-1,4-phthalazinedione ou l'un de ses sels pharmaceutiquement acceptables, une composition telle que définie dans l'une des revendications 4 ou 5 ou une combinaison telle que définie dans la revendication 6 pour utilisation dans la prophylaxie ou le traitement d'une insuffisance respiratoire aiguë causée par une infection à coronavirus par le SARS-CoV, le SARS-CoV-2 ou le MERS, dans laquelle ladite substance, composition ou combinaison est appliquée par voie parentérale sous forme d'injection intraveineuse, d'injection intra-artérielle ou d'injection intrapéritonéale.

10. 5-amino-2,3-dihydro-1,4-phthalazinedione ou l'un de ses sels pharmaceutiquement acceptables, une composition telle que définie dans l'une des revendications 4 ou 5 ou une combinaison telle que définie dans la revendication 6 pour utilisation dans la prophylaxie ou le traitement d'une insuffisance respiratoire aiguë causée par une infection à coronavirus par le SARS-CoV, le SARS-CoV-2 ou le MERS, dans laquelle ladite substance, composition ou combinaison est appliquée par inhalation à l'aide d'un nébuliseur à maille vibrante, d'un aérosol-doseur ou d'un inhalateur de poudres sèches.

11. 5-amino-2,3-dihydro-1,4-phthalazinedione ou l'un de ses sels pharmaceutiquement acceptables, une composition telle que définie dans l'une des revendications 4 ou 5 ou une combinaison telle que définie dans la revendication 6 pour utilisation dans la prophylaxie ou le traitement d'une insuffisance respiratoire aiguë causée par une infection à coronavirus par le SARS-CoV, le SARS-CoV-2 ou le MERS, dans laquelle ladite substance, composition ou combinaison est ajoutée à l'air de ventilation d'un dispositif de dérivation cardio-pulmonaire.

12. 5-amino-2,3-dihydro-1,4-phthalazinedione ou l'un de ses sels pharmaceutiquement acceptables, une composition telle que définie dans l'une des revendications 4 ou 5 ou une combinaison telle que définie dans la revendication 6 pour utilisation dans la prophylaxie ou le traitement d'une insuffisance respiratoire aiguë causée par une infection à coronavirus par le SARS-CoV, le SARS-CoV-2 ou le MERS, dans laquelle ladite substance, composition ou combinaison est appliquée sous forme de liposomes, de micelles, de vésicules multilamelares ou d'un complexe de cyclodextrine.
